# EUROPÄISCHE PATENTSCHRIFT

(11) **EP 1 123 289 B1**
(45) Veröffentlichungstag und Bekanntmachung des Hinweises auf die Patenterteilung: **27.11.2002**
(21) Anmeldenummer: 99953766.5
(22) Anmeldetag: 11.10.1999
(51) Int. Cl.: C07D 405/04, C09B 7/08, C09D 7/00

(54) **OXOBENZOFURANYLIDEN-DIHYDROINDOLONE**
OXOBENZOFURANYLIDE-DIHYDROINDOLONES
OXOBENZOFURANYLIDENE-DIHYDROINDOLONES

(30) Priorität: 22.10.1998 CH 213898
(43) Veröffentlichungstag der Anmeldung: 16.08.2001
(73) Patentinhaber: Ciba Specialty Chemicals Holding Inc., 4057 Basel (CH)
(72) Erfinder: NESVADBA, Peter, CH-1723 Marly (CH); JANDKE, Joachim, D-79585 Steinen (DE)
(86) Internationale Anmeldenummer: EP9907593
(87) Internationale Veröffentlichungsnummer: WO00024736

(56) Entgegenhaltungen:
- WO-A-99/13007
- C MARSCHALK: "Le diphtaloyl-6.7.6'.7' isoxindigo" BULLETIN DE LA SOCIETE CHIMIQUE DE FRANCE,FR,SOCIETE FRANCAISE DE CHIMIE. PARIS, Bd. 9, Nr. 5, 1. Januar 1942 (1942-01-01), Seiten 826-832, XP002090687 ISSN: 0037-8968
- D N NICOLAIDES ET AL: "Synthesis of alpha-Bromo substituted Ethyl (10-acetoxyphenanthren-9-y l)acetate" JOURNAL OF THE CHEMICAL SOCIETY, PERKIN TRANSACTIONS 1,GB,CHEMICAL SOCIETY. LETCHWORTH,1. Januar 1992 (1992-01-01), Seiten 2479-2484, XP002090686 ISSN: 0300-922X
- M. CH. MARSCHALK: "La phthaloyl-6,7 coumaranone et ses dérivés" BULLETIN DE LA SOCIETE CHIMIQUE DE FRANCE.,1942, Seiten 801-804, XP002133913 PARIS FR in der Anmeldung erwähnt
- R. STOLL ET AL.: "Über eine neue Darstellungsweise von Cumarandionen" BERICHTE DER DEUTSCHEN CHEMISCHEN GESELLSCHAFT, Bd. 54, 1921, Seiten 1213-1220, XP002133914 WEINHEIM DE in der Anmeldung erwähnt

## Beschreibung

Die vorliegende Erfindung betrifft Oxobenzofuranyliden-Dihydroindolone, Verfahren zu deren Herstellung sowie deren Verwendung.

In Bull.Soc.Chim.Fr., (1942), Seiten 801-804, wird ein trans-3-(2-Oxo-benzofuran-3-ylidene)-1,3-dihydro-indol-2-on (das Reaktionsprodukt von Phthaloyl-6,7-coumaron-2 und Isatin) und ein trans-2-(2-Oxo-benzofuran-3-ylidene)-1,2-dihydro-indol-3-on (das Reaktionsprodukt von Phthaloyl-6,7-cumaron-2 und α-Isatinanilid) beschrieben.

In Chem. Ber., Band 54, (1921), Seiten 1213-1220, werden zwei 2-(2-Oxo-benzofuran-3-ylidene)-1,2-dihydro-indol-3-one: zum einen das Kondensationsprodukt von 4,6-Dimethyl-cumarandion mit Indoxyl, und das Kondensationsprodukt von 5-Chlor-4,6-dimethylcumarandion mit Indoxyl beschrieben.

Nachteilig an den bekannten Verfahren ist die für grosstechnische und kommerzielle Zwecke zu aufwendige und zu teure Herstellung.

Der Erfindung lag daher die Aufgabe zugrunde, weitere Oxobenzofuranyliden-Dihydroindolone bereitzustellen, vorzugsweise sollten sie sich als Farb- oder Fluoreszensmittel, insbesondere zum Einfärben oder Pigmentieren von organischem oder anorganischem, hoch- oder niedermolekularem, insbesondere hochmolekularem organischen Material eignen. Ausserdem bestand die Aufgabe darin, ein wirtschaftliches Verfahren zur Herstellung von Oxobenzofuranyliden-Dihydroindolonen zu finden.

Demgemäss wurden Oxobenzofuranyliden-Dihydroindolone der Formeln trans-(Ia) und cis-(Ib) 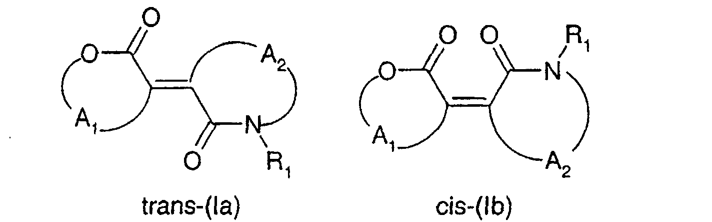 sowie der Formeln trans-(IIa) und cis-(llb) 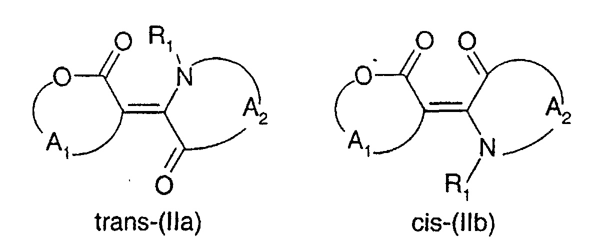 worin
A₁ und A₂ unabhängig voneinander unsubstituiertes oder 1- bis 4-fach substituiertes ortho-C₆-C₁₈-Arylen bedeuten, und
R₁ für Wasserstoff, C₁-C₂₅-Alkyl, C₅-C₁₂-Cycloalkyl, C₆-C₂₄-Aryl, einen heteroaromatischen Rest, -(CH₂)ₙ-COR₂ oder -(CH₂)ₘ-OR₃, steht,
worin R₂ für Hydroxy, -OX, unsubstituiertes oder ein- oder mehrfach mit Hydroxy, -OX, oder Amino substituiertes C₁-C₂₅-Alkoxy, C₁-C₂₅-Alkylamino oder C₁-C₂₅-Dialkylamino, Di-(C₆-C₂₄-Aryl)-amino, C₁-C₁₂-Alkyl, C₂-C₂₄-Alkenyl steht, und
X ein Kation bedeutet, und
R₃ für Wasserstoff oder -CO-(C₁-C₂₅-Alkyl) steht, und
n und m unabhängig voneinander für eine ganze Zahl im Bereich von 0 bis 6 stehen, und
eine C-C-Einfachbindung auch durch eine entsprechende Ethereinheit, C-O-C, ersetzt sein kann,
mit der Massgabe, dass, wenn R₁ für Wasserstoff und A₂ für 1,2-Phenylen stehen, A₁ nicht für 9,10-Anthrachinon-1,2-ylen, 4-Chlor-3,5-dimethyl-1,2-phenylen oder 3,5-Dimethyl-1,2-phenylen steht, gefunden.

Des weiteren wurden verbesserte Verfahren zur Herstellung von Oxobenzofuranyliden-Dihydroindolonen und deren Verwendung gefunden.

Erfindungsgemäss steht X für ein Kation eines Alkalimetalls wie Lithium, Natrium, Kalium,, Erdalkalimetalls wie Magnesium, Calcium, Strontium oder für Kupfer, Zink oder Aluminium stehen oder für ein quaternäres Amin wie [NR₄R₅R₆R₇]⁺, worin
R₄ und R₅ unabhängig voneinander für Wasserstoff, C₁-C₂₅Alkyl, C₆-C₁₈Aryl oder C₇-C₂₈Aralkyl stehen, und
R₆ und R₇ unabhängig voneinander für Wasserstoff, C₆-C₁₈Aryl, C₇-C₁₈Aralkyl, unsubstituiertes oder einfach oder mehrfach mit Halogen, Hydroxy oder C₁-C₁₂Alkoxy substituiertes C₁-C₂₅Alkyl oder C₂-C₂₄Alkenyl stehen, oder R₆ und R₇ zusammen mit dem gemeinsamen N für einen unsubstituierten oder mit C₁-C₄Alkyl einfach bis vierfach substituierten Pyrrolidin-, Piperidin-, Piperazin- oder Morpholin-, Carbazol-, Phenoxazin- oder Phenothiazin-Rest stehen.

In einer bevorzugten Ausführungsform stehen A₁ und A₂ unabhängig voneinander für ortho-C₆-C₁₈Arylen, welches beispielsweise substituiertes oder unsubstituiertes 1,2-Phenylen, 1,2-Naphthylen, 2,3-Naphthylen, 1,2-Phenanthrylen, 2,3-Phenanthrylen, 3,4-Phenanthrylen, 9,10-Phenanthrylen, 1,2-Anthracenyl, 2,3-Anthracenyl oder 1,2-Anthrachinonylen, 2,3-Anthrachinonylen, sein kann.

Als Substituenten für ortho-C₆-C₁₈Arylen kommen beispielsweise zweiwertige Reste wie 1,3-Butadien-1,4-ylen oder -CH=CH-NH-, oder ankondensierte substituierte oder unsubstituierte 5- oder 6-gliedrige Ringe in Betracht sowie gewünschtenfalls weitere Substituenten (siehe unten).

In einer besonders bevorzugten Ausführungsform steht A₁ für 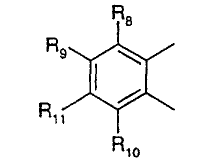 und A₂ für 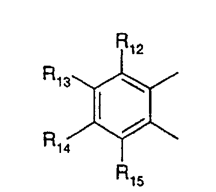 wobei R₈, R₉, R₁₁, R₁₀, R₁₂, R₁₃, R₁₄ und R₁₅ unabhängig voneinander Wasserstoff, Halogen, Cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂-Alkylen)-COOX, -O-(C₁-C₁₂-Alkylen)-COOH, -O-(C₁-C₁₂-Alkylen)-O-CO-R₁₇, -O-(C₁-C₁₂-Alkylen)-COOR₁₇, -O-(C₁-C₁₂-Alkylen)-CONR₁₉R₂₀, -O-(C₁-C₁₂-Alkylen)-OR₁₇, -(C₁-C₁₂-Alkylen)-O-CO-R₁₇, -(C₁-C₁₂-Alkylen)-OR₁₇, -COOR₁₇, -(C₁-C₁₂-Alkylen)-COOR₁₇, -(C₁-C₁₂-Alkylen)-CONR₁₉R₂₀, -(C₁-C₁₂-Alkylen)-COOX, -(C₁-C₁₂-Alkylen)-COOH, -COOX, -COOH, -SR₁₇, -SOR₁₇, -SO₂R₁₇, -SO₂NR₁₉R₂₀, -SO₃R₁₇, SO₃H oder SO₃X bedeuten,
worin
R₁₇, R₁₈, R₁₉ und R₂₀ unabhängig voneinander für Wasserstoff oder R₁₆ stehen, und
R₁₆ für unsubstituiertes oder einfach oder mehrfach mit Halogen, Hydroxy, Amino, Oxo, Carboxy, Cyano, -COOR₁₈ oder -COOX substituiertes C₁-C₂₅Alkyl, C₅-C₁₂-Cycioalkyl oder C₂-C₂₄Alkenyl, welches ununterbrochen oder einfach oder mehrfach durch O, S oder N-( C₁-C₂₅Alkyl), N-(C₂-C₂₄Alkenyl) unterbrochen sein kann, falls das Alkyl mehr als zwei und das Alkenyl mehr als drei Kohlenstoffatome aufweist, oder für unsubstituiertes oder einfach oder mehrfach mit Halogen, Nitro, Cyano, -OR₁₈, -SR₁₈,
-NR₁₉R₂₀, -CONR₁₉R₂₀, -COOR₁₈, -COOX, -COOH, -SO₂R₁₈, -SO₂NR₁₉R₂₀,-SO₃R₁₈, -SO₃X, -SO₃H, -NR₁₉COR₁₈ oder -NR₁₉COOR₁₈ substituiertes C₆-C₁₈Aryl, C₇-C₁₈Aralkyl oder Heteroaryl steht,
oder
R₁₉ und R₂₀ zusammen mit dem sie verbindendem Stickstoffatom für unsubstituiertes oder mit C₁-C₄Alkyl einfach bis vierfach substituiertes Pyrrolidinyl, Piperidinyl, Piperazinyl oder Morpholinyl, oder für Carbazolyl, Phenoxazinyl oder Phenothiazinyl stehen,
wobei gegebenenfalls jeweils R₈ und R₉, R₉ und R₁₁, R₁₁ und R₁₀ sowie R₁₂ und R₁₃, R₁₃ und R₁₄ oder R₁₄ und R₁₅ zusätzlich einen substituierten oder unsubstituierten 5- oder 6-gliedrigen Ring bilden können.

In einer weiteren besonders bevorzugten Ausführungsform steht mindestens einer der Substiuenten R₈, R₉, R₁₁, R₁₀, R₁₂, R₁₃, R₁₄ und R₁₅ für einen Rest, ausgewählt aus der Gruppe, bestehend aus den Resten Z₁, Z₂, Z₃ und Z₄ steht 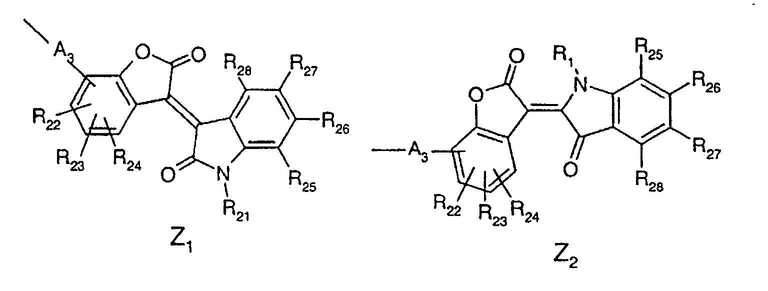 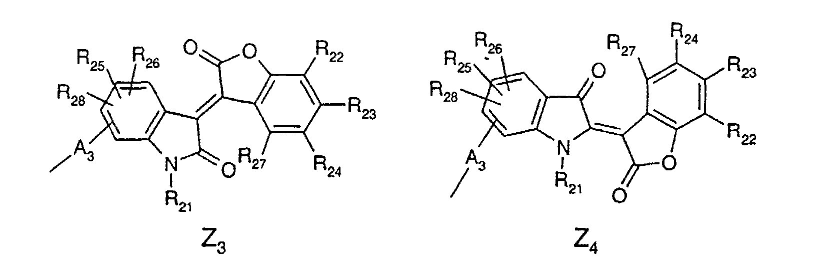 worin
R₂₂, R₂₃, R₂₄, R₂₅, R₂₆, R₂₇ und R₂₈ unabhängig voneinander für Wasserstoff, Halogen, Cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂-Alkylen)-COOX, -O-(C₁-C₁₂-Alkylen)-COOH, -O-(C₁-C₁₂-Alkylen)-O-CO-R17, -O-(C₁-C₁₂-Alkylen)-COOR₁₇, -O-(C₁-C₁₂-Alkylen)-CONR₁₉R₂₀, -O-(C₁-C₁₂-Alkylen)-OR₁₇, -(C₁-C₁₂-Alkylen)-O-CO-R₁₇, -(C₁-C₁₂-Alkylen)-OR₁₇, -COOR₁₇, -(C₁-C₁₂-Alkylen)-COOR₁₇, -(C₁-C₁₂-Alkylen)-CONR₁₉R₂₀, -(C₁-C₁₂-Alkylen)-COOX, -COOX, -COOH,-SR₁₇, -SOR₁₇, -SO₂R₁₇, -SO₂NR₁₉R₂₀, -SO₃R₁₇ oder SO₃X, SO₃H wobei die Reste R₁₇, R₁₈, R₁₉, R₂₀ die weiter oben angegebene Bedeutung haben, und
R₂₁ unabhängig von R₁ die gleiche Bedeutung wie R₁ besitzt, und
A₃ eine Einfachbindung, oder unsubstituiertes oder einfach oder mehrfach mit C₁-C₂₅Alkyl, C₆-C₂₄Aryl, Halogen, Hydroxy, -OX, Oxo, Cyano, -COOR₆, -COOX, -COOH, -SO₃R₆, -SO₃X, -SO₃H substituiertes C₁-C₂₄-Alkylen oder C₅-C₁₂-Cycloalkylen oder -OOC-(C₁-C₂₄-Alkylen)-COO-, -COO-(C₁-C₂₄-Alkylen)-OOC-, -NR₁₉CO-(C₁-C₂₄-Alkylen)-CONR₁₉-, -CONR₁₉-(C₁-C₂₄-Alkylen)-NR₁₉CO-; C₆-C₂₄Arylen oder Heteroarylen, bedeutet.

Eine weitere besonders bevorzugte Ausführungsform betrifft Oxobenzofuranyliden-Dihydroindolone, in denen A₁ für 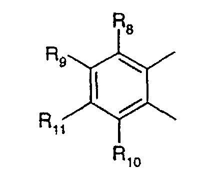 worin R₈, R₉, R₁₁ und R₁₀ die weiter oben angegebene Bedeutung haben, und A₂ für einen zweiwertigen Rest Z₅ oder Z₆ 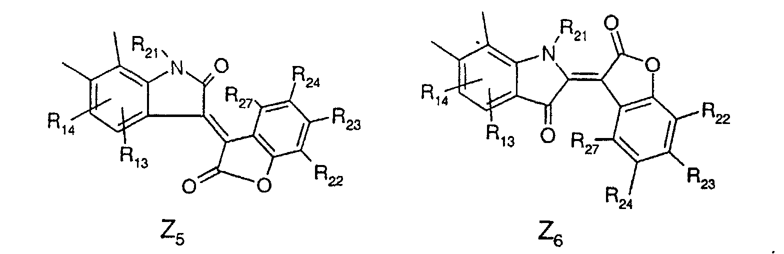 steht, wobei R₁₃, R₁₄, R₂₁, R₂₂, R₂₃, R₂₄ und R₂₇ die weiter oben angegebene Bedeutung haben.

Eine weitere bevorzugte Ausführungsform betrifft Oxobenzofuranyliden-Dihydroindolon der Formel (LI) 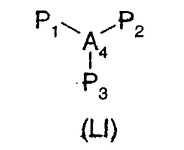 worin
P₁, P₂, P₃ unabhängig voneinander für jeweils einen Rest, ausgewählt aus der Gruppe, bestehend aus den Resten Z₁, Z₂, Z₃ und Z₄, und
A₄ für einen trivalenten Rest, vorzugsweise für 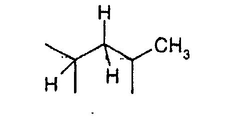 steht.

Die erfindungsgemässen Verbindungen können in verschiedenen Kristallmodifikationen vorkommen.

Alkyl, Alkenyl oder Alkylen kann geradkettig, verzweigt, monozyklisch oder polyzyklisch sein. Bevorzugt sind C₁-C₂₅Alkyl, C₂-C₂₄Alkenyl oder C₁-C₂₄Alkylen.

C₁-C₂₅Alkyl ist daher zum Beispiel ganz besonders bevorzugt C₁-C₄Alkyl wie Methyl, Ethyl, n-Propyl, Isopropyl, n-Butyl, sek.-Butyl, Isobutyl, tert.-Butyl, besonders bevorzugt C₁-C₆Alkyl, welches die für C₁-C₄Alkyl angegebene Bedeutung hat und zusätzlich für n-Pentyl, 2-Pentyl, 3-Pentyl, 2,2-Dimethylpropyl, n-Hexyl steht, und bevorzugt C₁-C₈Alkyl, welches die für C₁-C₆Alkyl angegebene Bedeutung besitzt und zusätzlich für beispielsweise n-Octyl, 1,1,3,3-Tetramethylbutyl und 2-Ethylhexyl steht sowie insbesondere C₁-C₁₂Alkyl, welches die für C₁-C₈Alkyl angegebene Bedeutung hat und zusätzlich für Decyl oder Dodecyl steht sowie C₁-C₂₅Alkyl, welches die für C₁-C₁₂Alkyl angegebene Bedeutung hat und zusätzlich Tetradecyl, Pentadecyl, Hexadecyl, Heptadecyl, Octadecyl, Eicosyl, Heneicosyl, Docosyl, Tetracosyl oder Pentacosyl bedeuten kann.

Als mono- oder polyzyklische Alkylreste seien beispielhaft genannt:
C₄-C₁₂Cycloalkyl wie Cyclobutyl, Cyclopentyl, Cyclohexyl, Cycloheptyl, Cyclooctyl, Cyclononyl, Cyclodecyl, Cycloundecyl oder Cyclododecyl, Trimethylcyclohexyl, Menthyl, Thujyl, Bornyl, 1-Adamantyl und 2-Adamantyl, insbesondere C₅-C₁₂Cycloalkyl wie Cyclopentyl, Cyclohexyl, Cycloheptyl, Cyclooctyl, Cyclononyl, Cyclodecyl, Cycloundecyl oder Cyclododecyl,
C₁-C₂₄Alkylen ist zum Beisplel Methylen, Ethylen, n-Propylen, Isopropylen, n-Butylen, sek.-Butylen, Isobutylen, tert.-Butylen, n-Pentylen, 2-Pentylen, 3-Pentylen, 2,2-Dimethylpropylen, n-Hexylen, n-Octylen, 1,1,3,3-Tetramethylbutylen, 2-Ethylhexylen, Nonylen, Decylen, Menthylen, Dodecylen, Tetradecylen, Hexadecylen, Heptadecylen, Octadecylen, Eicosylen, Heneicosylen, Docosylen oder Tetracosylen, bevorzugt C₁-C₁₂Alkylen wie Methylen, Ethylen, n-Propylen, Isopropylen, n-Butylen, sek.-Butylen, Isobutylen, tert.-Butylen, n-Pentylen, 2-Pentylen, 3-Pentylen, 2,2-Dimethylpropylen, n-Hexylen, n-Octylen, 1,1,3,3-Tetramethylbutylen, 2-Ethylhexylen, Nonylen, Decylen, besonders bevorzugt C₁-C₈Alkylen wie Methylen, Ethylen, n-Propylen, Isopropylen, n-Butylen, sek.-Butylen, Isobutylen, tert.-Butylen, n-Pentylen, 2-Pentylen, 3-Pentylen, 2,2-Dimethylpropylen, n-Hexylen, n-Octylen, 1,1,3,3-Tetramethylbutylen, 2-Ethylhexylen, ganz besonders bevorzugt C₁-C₆Alkylen wie Methylen, Ethylen, n-Propylen, Isopropylen, n-Butylen, sec-Butylen, Isobutylen, tert.-Butylen, n-Pentylen, 2-Pentylen, 3-Pentylen, 2,2-Dimethylpropylen, n-Hexylen und insbesondere C₁-C₄Alkylen wie Methylen, Ethylen, n-Propylen, Isopropylen, n-Butylen, sec-Butylen, Isobutylen, tert.-Butylen.

Als mono- oder polyzyklische Alkylenreste seien beispielhaft genannt:
C₄-C₁₂Cycloalkylen wie Cyclobutylen, Cyclopentylen, Cyclohexylen, Cycloheptylen, Cyclooctylen, Cyclononylen, Cyclodecylen, Cycloundecylen und Cyclododecylen, Trimethylcyclohexylen, Thujylen, Bornylen, 1-Adamantylen und 2-Adamantylen.
C₂-C₂₄Alkenyl steht zum Beispiel für Vinyl, Allyl, 2-Propen-2-yl, 2-Buten-1-yl, 3-Buten-1-yl, 1,3-Butadien-2-yl, 2-Penten-1-yl, 3-Penten-2-yl, 2-Methyl-1-buten-3-yl, 2-Methyl-3-buten-2-yl, 3-Methyl-2-buten-1-yl, 1,4-Pentadien-3-yl, 2,5-Hexadien-2-yl, oder die verschiedenen Isomeren von Hexenyl, Octenyl, Nonenyl, Decenyl, Dodecenyl, Tetradecenyl, Hexadecenyl, Heptadecenyl, Octadecenyl, Eicosenyl, Heneicosenyl, Docosenyl oder Tetracosenyl, wobei es insbesondere C₂-C₁₂Alkenyl wie Vinyl, Allyl, 2-Propen-2-yl, 2-Buten-1-yl, 3-Buten-1-yl, 1,3-Butadien-2-yl, 2-Penten-1-yl, 3-Penten-2-yl, 2-Methyl-1-buten-3-yl, 2-Methyl-3-buten-2-yl, 3-Methyl-2-buten-1-yl, 1,4-Pentadien-3-yl, 2,5-Hexadien-2-yl, oder die verschiedenen Isomeren von Hexenyl, Octenyl, Nonenyl, Decenyl, Dodecenyl bedeutet.

Als mono- oder polyzyklische Alkenylreste seien beispielhaft genannt:
C₄-C₁₂Cycloalkenyl wie 2-Cyclobuten-1-yl, 2-Cyclopenten-1-yl, 2-Cyclohexen-1-yl, 3-Cyclohexen-1-yl, 2,4-Cyclohexadien-1-yl sowie 1-*p*-Menthen-8-yl, 4(10)-Thujen-10-yl, 2-Norbornen-1-yl, 2,5-Norbornadien-1-yl, 7,7-Dimethyl-2,4-norcaradien-3-yl.
C₁-C₂₅Alkoxy ist -O-C₁-C₂₅Alkyl, bevorzugt -O-C₁-C₁₂Alkyl und besonders bevorzugt -O-C₁-C₄Alkyl, wobei die Alkylreste die oben angegebene Bedeutung haben.
C₁-C₂₅Alkylamino ist -NH-C₁-C₂₅Alkyl, bevorzugt -NH-C₁-C₁₂Alky und besonders bevorzugt -NH-C₁-C₄Alkyl, wobei die Alkylreste die oben angegebene Bedeutung haben.
C₁-C₂₅Dialkylamino ist -N-(C₁-C₂₅Alkyl)₂, bevorzugt -N-(C₁-C₁₂Alky)₂ und besonders bevorzugt -N-(C₁-C₄Alkyl)₂, wobei die Alkylreste die oben angegebene Bedeutung haben.
Di-(C₆-C₂₄Aryl)-amino ist (C₆-C₂₄Aryl)₂N- oder (C₆-C₁₂Aryl)₂N-, bevorzugt (C₆-C₁₂Aryl)₂N-.
C₆-C₂₄Aryl steht beispielsweise für Phenyl, 1-Naphthyl, 2-Naphthyl, 4-Biphenyl, Phenanthryl, 2- oder 9-Fluorenyl oder Anthracenyl, bevorzugt für C₆-C₁₂Aryl wie Phenyl, 1-Naphthyl, 2-Naphthyl, 4-Biphenyl.
Eine C-C-Einfachbindung kann auch durch eine entsprechende Ethereinheit ersetzt sein wie C-O-C, beispielsweise bei C₄Alkyl kann man -CH₂-CH₂-O-CH₂-CH₃ erhalten oder bei C₆Alkyl -CH₂-CH₂-O-CH₂-CH₂-O-CH₂-CH₃.

Mit Oxo substituiertes C₁-C₁₂Alkyl bedeutet, dass wenigstens eine Methylengruppe durch eine Carbonylgruppe ersetzt ist, beispielsweise bei C₂Alkyl -C(=O)-CH₃.

Alkyl kann auch sowohl mit einer Oxo-Einheit versehen als auch durch eine -O-Einheit unterbrochen sein (Voraussetzung hierfür ist selbstverständlich die Anwesenheit von mindestens zwei C-Atomen in der Kette), beispielsweise -(CH₂)₃-O-C(=O)-C(CH₃)₃, -C(=O)-(CH₂)₆-OCH₃ oder -C(CH₃)₂-COO-(CH₂)₃-CH₃, -C(=O)-(CH₂)₆-OCH₂- oder -C(CH₃)₂-COO-(CH₂)₃-CH₂-, oder bevorzugt beispielsweise -O-(C₁-C₆Alkylen)-COO(C₁-C₆Alkyl) wie -O-CH₂-COOCH₃, -O-(CH₂)₂-COOCH₃, -O-(CH₂)₃-COOCH₃ oder -O-(C₁-C₆Alkylen)-COOH wie -O-CH₂-COOH oder -O-(CH₂)₂-COOH.

C₇-C₂₈Aralkyl steht beispielsweise für Benzyl, 2-Benzyl-2-propyl, β-Phenyl-ethyl, α,α-Dimethylbenzyl, ω-Phenyl-butyl, ω,ω-Dimethyl-ω-phenyl-butyl, ω-Phenyl-dodecyl, ω-Phenyl-octadecyl, ω-Phenyl-eicosyl oder ω-Phenyl-docosyl, bevorzugt für C₇-C₁₈Aralkyl wie für Benzyl, 2-Benzyl-2-propyl, β-Phenyl-ethyl, α,α-Dimethylbenzyl, ω-Phenyl-butyl, ω,ω-Dimethyl-ω-phenyl-butyl, ω-Phenyl-dodecyl oder ω-Phenyl-octadecyl, und besonders bevorzugt für C₇-C₁₂Aralkyl wie Benzyl, 2-Benzyl-2-propyl, β-Phenyl-ethyl, α,α-Dimethylbenzyl, ω-Phenyl-butyl, ω,ω-Dimethyl-ω-phenyl-butyl oder ω-Phenyl-dodecyl.

Als Heteroaryl kann man einen mehrfach ungesättigten heterozyklischen Rest aus 5 bis 18 Atomen, ausgewählt aus der Gruppe bestehend aus C, N, O und S, welches mindestens 6 konjugierte π-Elektronen enthält, einsetzen, zum Beispiel Thienyl, Benzo[b]thienyl, Dibenzo[b,d]thienyl, Thianthrenyl, Furyl, Furfuryl, 2H-Pyranyl, Benzofuranyl, Isobenzofuranyl, Dibenzofuranyl, Phenoxythiinyl, Pyrrolyl, Imidazolyl, Pyrazolyl, Pyridyl, Bipyridyl, Triazinyl, Pyrimidinyl, Pyrazinyl, Pyridazinyl, Indolizinyl, Isoindolyl, Indolyl, Indazolyl, Purinyl, Quinolizinyl, Chinolyl, Isochinolyl, Phthalazinyl, Naphthyridinyl, Chinoxalinyl, Chinazolinyl, Cinnolinyl, Pteridinyl, Carbazolyl, Carbolinyl, Benzotriazolyl, Benzoxazolyl, Phenanthridinyl, Acridinyl, Perimidinyl, Phenanthrolinyl, Phenazinyl, Isothiazolyl, Phenothiazinyl, Isoxazolyl, Furazanyl oder Phenoxazinyl, vorzugsweise die oben genannten mono- oder bicyclischen heteroaromatischen Reste.

Halogen oder Hal steht für Chlor, Brom, Fluor oder lod, bevorzugt für Fluor oder Chlor.

Mit Halogen, Hydroxy, C₁-C₁₂Alkoxy oder Cyano einfach oder mehrfach substituiertes C₁-C₁₂Alkyl oder C₂-C₁₂Alkenyl ist beispielsweise 2-Chlor-ethyl, Trifluormethyl, Pentafluorethyl, β,β,β-Trifluorethyl, Trichlorvinyl, ω-Chlor-propyl, ω-Brom-butyl, Perfluorhexyl, Perfluordodecyl, 2-Hydroxy-ethyl, 2-Methoxy-ethyl, 2-Ethoxy-ethyl, 2-Butoxy-ethyl, 2,3-Dihydroxypropyl, 2,3-Dimethoxy-propyl, 2,3-Dimethoxy-propyl oder 2-Cyano-ethyl, bevorzugt Trifluormethyl, 2-Hydroxy-ethyl, 2-Methoxy-ethyl, 2-Ethoxy-ethyl oder 2-Cyano-ethyl.

Besonders bevorzugte Oxobenzofuranyliden-Dihydroindolone sind Verbindungen der Formeln 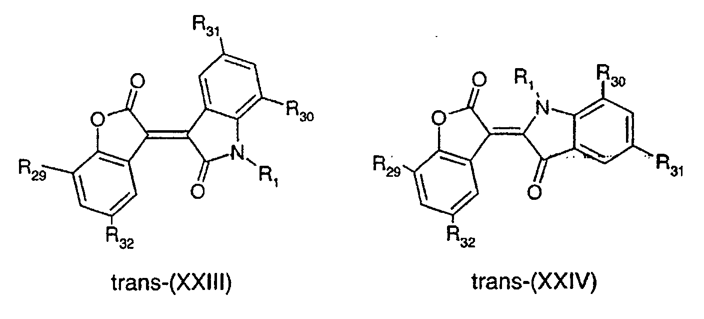 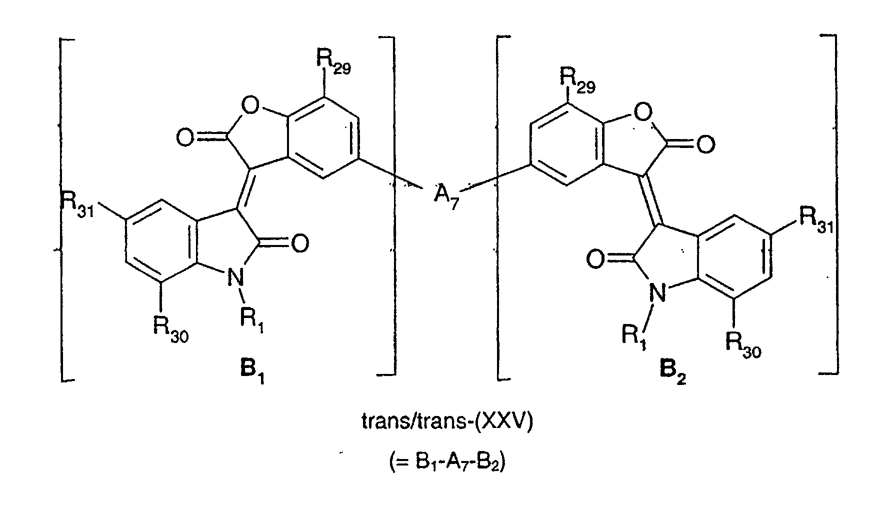 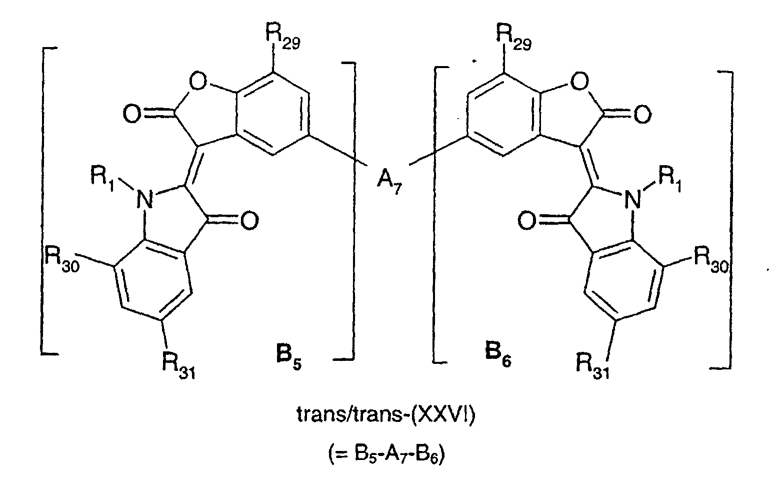 worin
R₂₉, R₃₂, R₃₀ und R₃₁ unabhängig voneinander Wasserstoff, Halogen, -NO₂, R₃₃, -OR₃₄, -SR_{34,} insbesondere OC₉-C₁₈Alkyl oder SC₉-C₁₈Alkyl, -O-(C₁-C₁₂-Alkylen)-COOX,--O-( C₁-C₁₂-Alkylen)-COOH,-O-(C₁-C₁₂-Alkylen)-COOR₃₄, -O-(C₁-C₁₂-Alkylen)-O-CO-R₃₄, (C₁-C₁₂-Alkylen)-O-CO-R₃₄, -O-( C₁-C₁₂-Alkylen)-OR₃₄, -(C₁-C₁₂-Alkylen)-OR₃₄, -COOR₃₄,-(C₁-C₁₂-Alkylen)-COOR₃₄, -(C₁-C₁₂-Alkylen)-CONR₃₅R₃₆, -(C₁-C₁₂-Alkylen)-COOX, -(C₁-C₁₂-Alkylen)-COOH
worin
R₃₃ für ein unsubstituiertes oder einfach oder mehrfach mit Oxo, Cyano oder COOX, COOH substituiertes C₁-C₂₅Alkyl, welches ununterbrochen oder einfach oder mehrfach durch O unterbrochen sein kann, oder für unsubstituiertes oder einfach oder mehrfach mit Halogen, Nitro, Cyano, OR₃₅, NR₃₅R₃₆, CONR₃₅R₃₆, NR₃₇COR₃₅ oder NR₃₇COOR₃₅ substituiertes C₆-C₁₀Aryl oder C₇-C₁₀Aralkyl steht;
R₃₄, R₃₅, R₃₆ Wasserstoff oder R₃₃ bedeutet,
und
A₇ für eine Einfachbindung, C₁-C₂₄-Alkylen oder C₅-C₁₂-Cycloalkylen steht.

Ganz besonders bevorzugt sind Verbindungen der Formeln 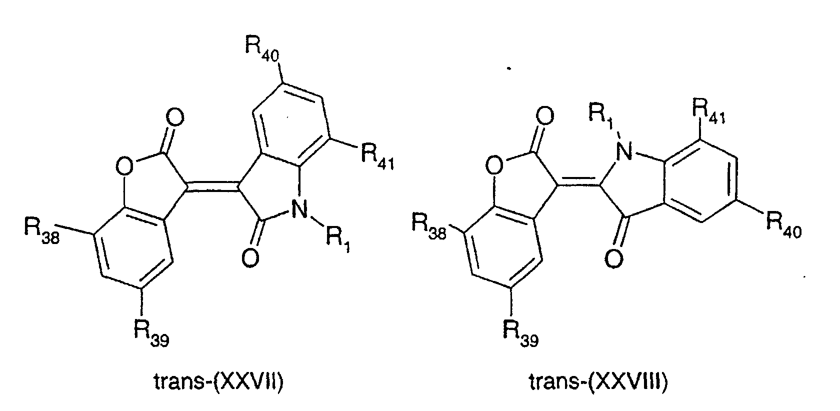 worin
R₃₈, R₃₉, R₄₁ oder R₄₀, unabhängig voneinander Wasserstoff, Chlor oder Brom, R₄₂ -OR₄₂, -SR₄₂, insbesondere -OC₁-C₁₈Alkyl oder -SC₁-C₁₈Alkyl sind, worin
R₄₂ für unsubstituiertes oder einfach oder mehrfach mit Oxo, Cyano, -COOH, -COOC₁-C₁₈Alkyl oder -COOX substituiertes C₁-C₂₅Alkyl, welches ununterbrochen oder einfach oder mehrfach durch O unterbrochen sein kann (mit der Voraussetzung, dass mehr als ein C-Atom vorhanden ist), wie insbesondere -O-CH₂COOCH₃, oder für C₆-C₁₀Aryl oder C₇-C₁₀Aralkyl, steht.

Des weiteren ganz besonders bevorzugt ist eine Verbindung der Formel (XXVII), worin R₃₈ tert.-Butyl und R₃₉ -OMe und R₄₁ und R₄₀ Wasserstoff bedeuten.

In der vorliegenden Erfindung wurden auch cis und trans Bisbenzofuranon-Derivate der Oxobenzofuranyliden-Dihydroindolone gefunden, wie 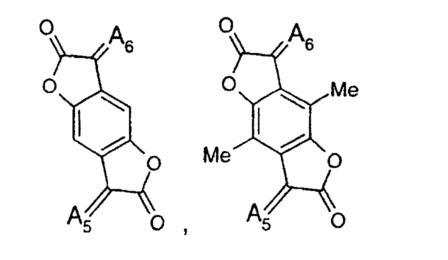 worin

A₅ und A₆ unabhängig voneinander für jeweils zwei Wasserstoffe stehen, unter der Massgabe, dass sie nicht gleichzeitig für zwei Wasserstoffe stehen; des weiteren stehen sie unabhängig voneinander für einen substituierten oder unsubstituierten Isatin-Rest

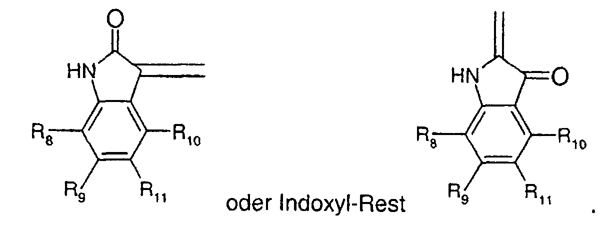

Die obigen Bisbenzofuranon-Derivate der Oxobenzofuranyliden-Dihydroindolone sind nach den erfindungsgemässen Verfahren zur Herstellung der Oxobenzofuranyliden-Dihydroindolone zugänglich. Beispielsweise kann man sie herstellen, indem man Bisbenzofuranone 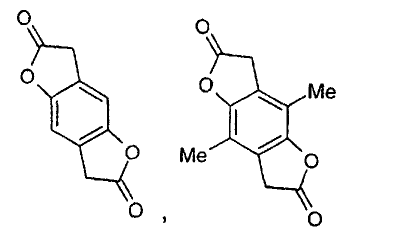 mit 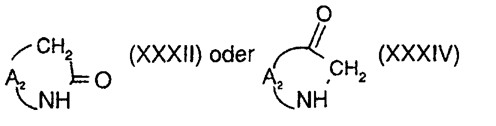 zur Reaktion bringt.

Die vorliegende Erfindung betrifft des weiteren Zusammensetzungen, enthaltend mindestens ein erfindungsgemässes Oxobenzofuranyliden-Dihydroindolon sowie gewünschtenfalls weitere Oxobenzofuranyliden-Dihydroindolone.

Eine besonders bevorzugte Ausführungsform betrifft Zusammensetzungen mit zwei Oxobenzofuranyliden-Dihydroindolonen, bevorzugt zwei Verbindungen, ausgewählt aus der Gruppe, bestehend aus den erfindungsgemässen Verbindungen und den vier eingangs zitierten Verbindungen des Standes der Technik (trans-3-(2-Oxo-benzofuran-3-ylidene)-1,3-dihydro-indol-2-on, trans-2-(2-Oxo-benzofuran-3-ylidene)-1,2-dihydro-indol-3-on, das Kondensationsprodukt von 4,6-Dimethyl-cumarandion mit Indoxyl, und das Kondensationsprodukt von 5-Chlor-4,6-dimethyl-cumarandion mit Indoxyl.

Das Molverhältnis der Oxobenzofuranyliden-Dihydroindolonen in den Zusammensetzungen wählt man üblicherweise im Bereich von 99:1 bis 1:99.

Eine weitere Ausführungsform betrifft Zusammensetzungen mit drei Oxobenzofuranyliden-Dihydroindolonen, bevorzugt mit mindestens einer erfindungsgemässen Verbindung. Hierbei wählt man das Molverhältnis üblicherweise im Bereich von 98:1:1 bis 1:98:1 oder 1:1:98, insbesondere 25:50:25, wobei die Summen der Molverhältnisse sich immer zu 100 ergänzen.

Die erfindungsgemässen Zusammensetzungen können nach an sich bekannten Methoden des Vermischens aus den Einzelverbindungen, oder durch Mischsynthese hergestellt werden (s.u.).

Als besonders bevorzugte erfindungsgemässe Verbindungen seien die folgenden genannt:

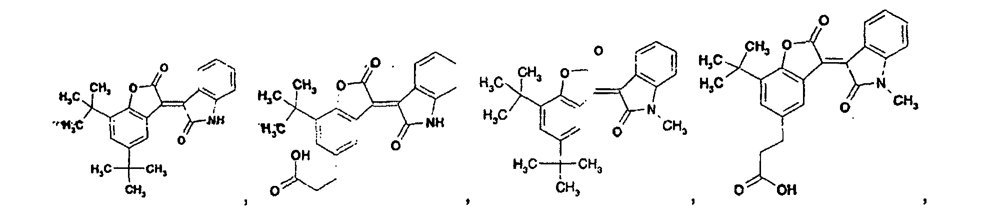

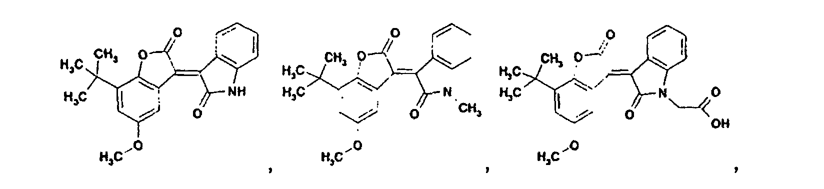

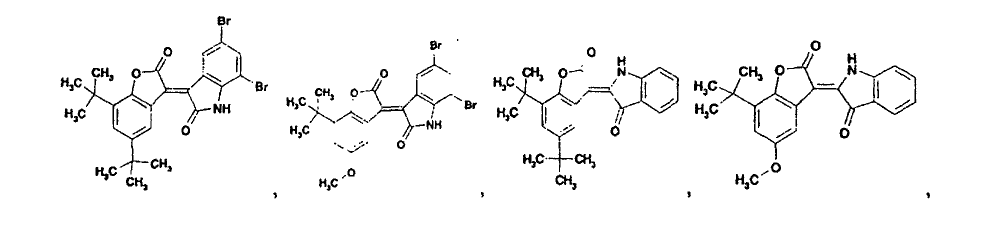

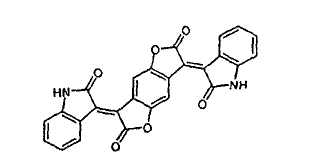

Die erfindungsgemässen Verbindungen kann man in Analogie zum in Bull.Soc.Chim.Fr, (1942), Seiten 801-804 und 826-832, beschriebenen Verfahren herstellen.

Die vorliegende Erfindung betrifft des weiteren ein Verfahren zur Herstellung der Oxobenzofuranyliden-Dihydroindolone der Formeln trans-(Ia) und cis-(Ib) durch Umsetzung eines Benzofuranon-oder Bisbenzofuranon-Derivats mit einem lsatin-Derivat in Gegenwart eines sauren oder basischen Katalysators, indem man eine Verbindung der Formel (XXIX)

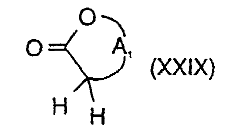

in der A₁ die weiter oben angegebene Bedeutung hat, mit einer Verbindung der Formel (XXX) oder (XXXa), 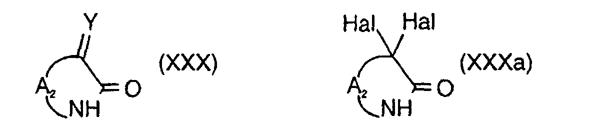 worin
Y für O, S, NH, N-(C₁-C₂₄Alkyl), N-(C₆-C₁₈Aryl) oder N-(C₇-C₂₈Aralkyl) und
Hal für Halogen stehen, und
A₂ die weiter oben angegebene Bedeutung hat, umsetzt.

Anstelle der Carbonylverbindungen (XXX) (Y = 0) kann man selbstverständlich auch entsprechende "maskierte" Carbonylverbindungen (im folgenden auch als synthetische Äquivalente bezeichnet) wie Schiffsche Basen u.a. einsetzen. Dies gilt auch für alle weiter unten beschriebenen Verfahren.

Eine weitere Ausführungsform betrifft ein Verfahren zur Herstellung der Oxobenzofuranyliden-Dihydroindolone der Formeln trans-(Ia) und cis-(Ib) durch Umsetzung eines Benzofuranon- oder Bisbenzofuranon-Derivats mit einem Oxindol-Derivat in Gegenwart eines sauren oder basischen Katalysators, indem man eine Verbindung der Formel (XXXI) oder (XXXIa) 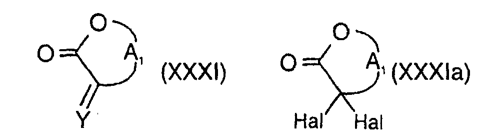 mit einer Verbindung der Formel (XXXII) 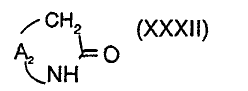 in der A₂, A₁, Y und Hal die weiter oben angegebene Bedeutung haben, umsetzt. Eine weitere Ausführungsform betrifft ein Verfahren zur Herstellung von Verbindungen der Formeln trans-(IIa) und cis-(IIb) durch Umsetzung eines Benzofuranon- oder Bisbenzofuranon-Derivats mit einem Indoxyl-Derivat in Gegenwart einer Säure oder einer Base, indem man eine Verbindung der Formeln (XXXI) oder (XXXIa) mit einer Verbindung der Formel (XXXIV) 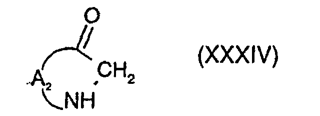 worin A₂ die weiter oben angegebene Bedeutung hat,
umsetzt.

Eine weitere Ausführungsform betrifft en Verfahren zur Herstellung von Verbindungen der Formeln trans-(Ia), trans-(IIa) oder cis-(Ib), cis-(IIb) durch Umsetzung eines Benzofuranon-oder Bisbenzofuranon-Derivats mit einem Indoxyl- oder Isatin-Derivat in Gegenwart einer Säure oder einer Base, indem man eine Verbindung der Formel (XXXI) oder (XXXIa) mit
(a) einem Imin der Formel (XXXV) 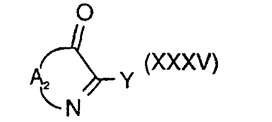 in der A₂ und Y die weiter oben angegebenen Bedeutungen haben,
   oder
(b) einem Ketal der Formeln (XXXVI) oder (XXXVII) 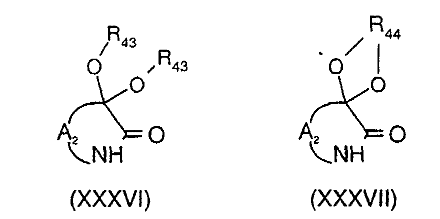
worin
R₄₃ C₁-C₆Alkyl und R₄₄ einen unsubstituierten oder substituierten bivalenten C₁-C₆-Alkylen-Rest wie beispielsweise -CH₂-CH₂-CH₂-, -CH₂-C(CH₃)₂-CH₂-, -CH₂-CH₂-CH₂-CH₂-, -CH₂-CH₂-CH₂-CH₂-, bedeuten,
umsetzt.

Bei allen erfindungsgemässen Verfahren handelt es sich um chemisch ähnliche Umsetzungen, die den Kondensationsreaktionen sogenannter C-H-acider Verbindungen mit einem Elektrophil entsprechen und untenstehend gemeinsam beschrieben werden.

Die vorliegende Erfindung betrifft des weiteren Mischsynthesen gemäss den erfindungsgemässen Verfahren zur Herstellung der erfindungsgemässen Zusammensetzungen, indem man
Mischungen von unterschiedlichen C-H-aciden Verbindungen der Formeln (XXIX), (XXXIV) oder (XXXII) mit einer Carbonylverbindung oder deren synthetischen Äquivaienten der Formeln (XXX), (XXXa) oder (XXXI), (XXXIa)
oder
Mischungen von unterschiedlichen C-H-aciden Verbindungen der Formeln (XXIX), (XXXIV) oder (XXXII) mit einem Imin der Formel (XXXV) oder Ketal der Formeln (XXXVI), (XXXVII) oder
Mischungen von verschieden substituierten Carbonylverbindungen oder deren synthetischen Äquivalenten der Formeln (XXX), (XXXa) oder (XXXI), (XXXIa) mit einer C-H-aciden Komponte der Formeln (XXIX), (XXXIV) oder (XXXII)
oder
Mischungen von unterschiedlichen Iminen der Formel (XXXV) oder Ketalen der Formeln (XXXVI), (XXXVII) mit einer C-H-aciden Komponente der Formeln (XXIX), (XXXIV) oder (XXXII) umsetzt.

Üblicherweise setzt man die Reaktionen in Gang, indem man die eine Reaktionskomponente zur anderen gibt. Die Reihenfolge der Zugabe spielt für die erfindungsgemässen Reaktionen in der Regel keine Rolle.

Das Molverhältnis der Carbonylverbindung oder deren synthetischem Äquivalent der Formeln (XXIX), (XXX), (XXXa) oder (XXXI), (XXXIa) oder einem lmin der Formel (XXXV) oder Ketal der Formel (XXXVII) zur CH-aciden Verbindung wählt man üblicherweise im Bereich von 1,5:1 bis 0,5:1, bevorzugt im Bereich von 1,2:1 bis 0,8:1 und ganz besonders bevorzugt beträgt es 1:1.

Das Molverhältnis des Bisbenzofuranons zum Isatin liegt im allgemeinen im Bereich von 1:5 bis 1:1,5 und bevorzugt im Bereich von 1:3 bis 1:2.

Es hat sich als vorteilhaft erwiesen, die Umsetzung bei erhöhter Temperatur durchzuführen, insbesondere im Bereich bei der Siedetemperatur des Reaktionsgemisches, beispielsweise im Bereich von 20 bis 200°C, bevorzugt von 60 bis 150°C.

Üblicherweise führt man die Umsetzung in Gegenwart von organischen Lösungsmitteln durch. Als organische Lösungsmittel kommen beispielsweise Alkohole wie (C₁-C₂₅Alkyl)-OH, insbesondere Ethanol, Methanol, Isopropanol oder Säuren wie (C₁-C₂₅Alkyl)-COOH, insbesondere Essigsäure, oder Anhydride wie (C₁-C₂₅Alkyl)-COOCO-(C₁-C₂₅Alkyl) sowie dipolar-aprotische Lösungsmittel wie Acetonitril, Benzonitril, N,N'-Dimmethylformamid, N,N'-Dimethylacetamid, Nitrobenzol, N-Methylpyrrolidon, gewünschtenfalls halogenierte aliphatische oder aromatische Kohlenwasserstoffe oder deren Gemische wie Benzin (als Gemisch verschiedener, im wesentlichen aliphatischer Kohlenwasserstoffe), Trichlorethan, Benzol oder unsubstituiertes oder mit Alkyl, Alkoxy oder Halogen substituiertes Benzol wie Toluol, Xylol, Anisol oder Chlorbenzol, oder Ester wie Ethylacetat, Butylacetat oder Ether wie zum Beispiel Tetrahydrofuran, Dioxan, EtOCH₂CH₂OH (z.B. als Cellosolve® von Fluka im Handel) oder Glykolether wie Ethylenglycolmethylether, Ethylenglycolethylether, Diethylenglycolmonomethylether oder Diethylenglycolmonoethylether, oder stickstoffhaltige Lösungsmittel wie Pyridin, Triethylamin, Picolin oder Chinolin, in Betracht. Die oben erwähnten Lösungsmittel können auch als Mischungen untereinander verwendet werden.

1 bis 20 Gew.-% Lösungsmittel werden üblicherweise auf ein Gew.-% der Summe der Reaktanden eingesetzt.

Für die Herstellung von Verbindungen der Formeln (la) oder (Ib) hat es sich als besonders vorteilhaft erwiesen, in alkoholischer oder essigsaurer Lösung im Bereich der Siedetemperatur des Reaktionsgemisches zu arbeiten, beispielsweise im Bereich von 100 bis 130°C.

Besonders bevorzugt ist die Umsetzung in Gegenwart einer zusätzlichen organischen oder anorganischen Base oder Säure, insbesondere in Gegenwart von katalytischen Mengen einer Base oder Säure. Beispielsweise wählt man die Menge an Base oder Säure im Bereich von 0,1 bis 10, bevorzugt von 1 bis 5 Mol-%, bezogen auf die Molsumme der Ausgangsstoffe.

Als anorganische Basen kommen beispielsweise Alkalimetalle wie Lithium, Natrium oder Kalium sowie deren Hydroxide und Carbonate wie Lithium-, Natrium- oder Kaliumhydroxid oder Lithium-, Natrium- oder Kaliumcarbonat, oder Alkalimetallamide wie Lithium-, Natriumoder Kaliumamid oder Alkalimetallhydride wie Lithium-, Natrium- oder Kaliumhydrid oder Erdalkalimetall- oder Alkalimetallalkoholate, aus primären, sekundären oder tertiären aliphatischen C₁-C₁₀-Alkyl-Alkoholen, wie Lithium-, Natrium- oder Kaliummethylat, -ethylat,-n-propylat, -isopropylat, -n-butylat, -sek.-butylat, -tert.-butylat, -2-methyl-2-butylat, -2-methyl-2-pentylat,-isopropylat,-n-butylat, sek.-butylat, -tert.-butylat, -2-methyl-2-butylat, -2-methyl-2-pentylat, -3-methyl-3-pentylat, -3-ethyl-3-pentylat, und weitere organische aliphatische, aromatische oder heteroaromatische Stickstoffbasen, insbesondere Diazabicyclooctan, Diazabicycloundecan und 4-Dimethylaminopyridin und Trialkylamin wie Trimethyl- oder Triethylamin in Frage. Die oben erwähnten Basen können auch als Mischungen untereinander verwendet werden.

Als anorganische Säuren kommen beispielsweise Salzsäure, Schwefelsäure oder Bortrifluorid in Betracht.

Als organische Säuren verwendet man zum Beispiel organische Sulfonsäuren oder Carbonsäuren wie unsubstituierte oder substituierte aliphatische, cycloaliphatische, carbocyclisch-aromatische, heterocyclisch-aromatische oder araliphatische Sulfon- oder Carbonsäuren.

Beispielsweise kann man als aliphatische Sulfonsäuren Methan-, Ethan-, n-Propan- oder Hexansulfonsäure oder deren entsprechende mit Fluor substituierte Derivate einsetzen.

Aliphatische Carbonsäuren bedeuten beispielsweise Ameisen-, Essig-, Propion-, Butyr-, Pivalin-, Capron-, 2-Ethylhexylcarboxylsäuren oder Fettsäuren wie Laurylsäure, Myricetinoder Stearinsäure sowie deren mit Fluor substituierte Derivate.

Beispiele für cycloaliphatische Sulfon- oder Carbonsäuren sind Cyclohexancarbonsäuren, Campher-10-sulfonsäure oder deren mit Fluor substituierte Derivate.

Beispiele für carbocyclische-aromatische Sulfonsäuren sind Benzol-, Toluol-, Ethylbenzol-, Isopropylbenzol-, Dodecylbenzol- oder Dimethylbenzolsulfonsäuren, 2,4,6-triisopropylbenzolsulfonsäure, 2,4;6-Trimethylbenzolsulfonsäure, Naphthalinsulfonsäure, -disulfonsäure oder -trisulfonsäure und deren entsprechend mit Alkyl oder Fluor substituierten Derivate.

Beispiele für heterocyclische-aromatische Sulfonsäuren sind Pyridin-, Thiophen- oder Pyrrolsulfonsäuren sowie deren entsprechend mit Fluor substituierte Derivate.

Beispiele für carbocyclische-aromatische Carbonsäuren sind Benzoesäure, Toluol-, Ethylbenzol-, Isopropylbenzol- oder Dimethylbenzolcarbonsäure, Naphthalincarbonsäure oder Anthracencarbonsäure und deren entsprechend mit Fluor substituierte Derivate. Beispiele für araliphatische Carbonsäuren sind Benzylcarbonsäuren, α-Methylbenzylcarbonsäuren und Cinnamylsäure sowie deren entsprechend mit Fluor substituierten Derivate.

Ferner wird in einer weiteren Ausgestaltung des erfindungsgemässen Verfahrens eine C-Hacide Verbindungen mit Ketalen in Gegenwart einer Base und anschliessend einer Säure umgesetzt. Die Rolle des Ketals ist es üblicherweise, die 3-Carbonylgruppe temporär während der basich katalysierten selektiven Kondensation an der 2-Carbonylgruppe gegen Kondensation zu schützen. Nach der Kondensation wird das Ketal im allgemeinen mit Säure abgespalten und die Carbonylgruppe freigesetzt.

Die Aufarbeitung und Isolation der erfindungsgemässen Verbindungen erfolgt in der Regel nach Methoden, die dem Fachmann allgemein bekannt sind. Üblicherweise filtriert man das ausgefallene Rohprodukt und wäscht den Filterrückstand mit vorzugsweise einem Lösungsmittel, in dem das Reaktionsprodukt nur schlecht löslich ist. Gewünschtenfalls kann man das Rohprodukt vor der Isolation noch Reinigungsoperationen unterziehen. Hierzu kann man beispielsweise die organische Phase, enthaltend das Reaktionsprodukt, mit Wasser, insbesondere salzsaurem Wasser oder einer Natriumhydroxidlösung, waschen und anschliessend die organische Phase isolieren und aufkonzentrieren, bevorzugt bis zur Trockene. In einer weiteren Variante der Aufarbeitung kann man das organische Reaktionsprodukt auch direkt eindampfen und anschliessend beispielsweise durch Umkristallisation oder säulenchromatographische Trennung reinigen. Die Isolation erfolgt im Falle der Umkristallisation üblicherweise durch Filtration und anschliessender Wäsche des Filterrückstandes mit vorzugsweise einem Lösungsmittel, in dem das Reaktionsprodukt nur schlecht löslich ist. Die säulenchromatographierte organische Phase enthaltend das Reaktionsprodukt kann direkt eingedampft werden. Gewünschtenfalls können die Reaktionsprodukte nach der Isolation getrocknet werden. Hierfür benutzt man in der Regel allgemein bekannte Trocknungsapparate wie Trockenschränke oder Schaufeltrockner.

Für die Herstellung von Verbindungen der Formeln (IIa) oder (IIb) hat es sich als besonders vorteilhaft erwiesen, in Gegenwart von Säuren oder Anhydriden wie Essigsäure oder Essigsäureanhydrid zu arbeiten.

Die Edukte für dieses Verfahren werden in Analogie zu bekannten Verfahren hergestellt. Isatin-Verbindungen können nach aligemein bekannten Methoden wie zum Beispiel von W. C. Sumpter in Chem.Rev. 34, 413, 1944, beschrieben hergestellt werden. Die N-Alkylierung von Isatinen ist ebenfalls allgemein bekannt und beispielsweise von O. M. Radul et al. in Khimiya Geterotsiklicheskikh Soedinenii 353, 1983, beschrieben.

Die Herstellung der Oxobenzofuranyl-Verbindungen ist ebenfalls allgemein bekannt oder kann in Analogie zu bekannten Methoden der Herstellung von in 3-Stellung unsubstituierten Furanon und 3-Oxo-Furanonverbindungen erfolgen. In 3-Stellung unsubstituierte Furanone können beispielsweise analog dem Verfahren von H.-D. Becker, K. Gustafsson, J.Org.Chem.42, 2966 (1977) aus Phenolen durch Umsetzung mit Glyoxal hergestellt werden.

Bisbenzofuranone sind bekannt und werden zum Beispiel nach J.H. Wood, L. Cox, Org. Synth. III, 286 (1955) hergestellt, oder (4,8- Dimethyl-3,7-dihydro-benzo(1,2-b-4,5-b')difuran-2,6-dione wird beispielsweise nach L.I. Smith, J. Nichols, J. Am. Chem. Soc., 65, 1739 (1943) hergestellt.

3-Oxo-Furanonverbindungen können durch Oxidation von in 3-Stellung unsubstituierten Furanonverbindungen, oder durch Oxidation von 3-Hydroxy-Furanonverbindungen nach allgemein bekannten Methoden zur Oxidation von Hydroxy- zu Keto-Verbindungen hergestellt werden. Diese sind beispielsweise in Houben-Weyl, Methoden der Organischen Chemie, 4. Auflage, Band 4/1a & 4/1b beschrieben. In J. Org. Chem., 56, Seite 6110 (1991), von Z- Ma, J.M. Bobbitt ist die Oxidation mit Nitroxiden beschrieben. 3-Hydroxy-Furanonverbindungen können in Analogie zu dem Verfahren von 3-Hydroxy-Benzofuranonen, das in US 5,614,572 beschrieben ist, hergestellt werden. Des weiteren können 3-Oxo-Furanonverbindungen in Analogie zum Verfahren von D.J. Zwaneburg und W.A.P. Reyen, das in Synthesis, 624, von 1976 beschrieben ist, hergestellt werden.

Eine weitere Ausführungsform betrifft die Herstellung der erfindungsgemässen Bis-(Oxobenzofuranyliden-Dihydroindolone) aus Bis-(Furanonverbindungen) der Formel (XXXVIII) 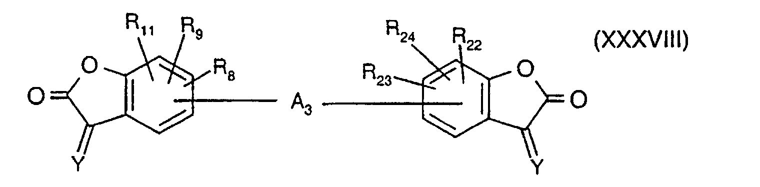 oder der Formel (XXXIX) 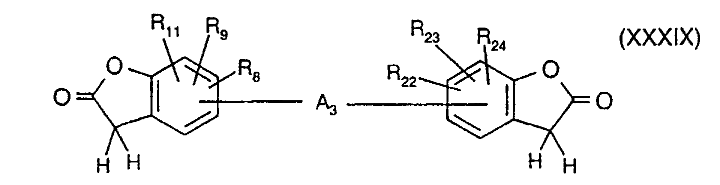 hergestellt, oder aus entsprechenden Bis-lsatin- oder Indoxylverbindungen oder Mischungen davon.

In Analogie zur Herstellung der Bis-Verbindungen können die Tris- oder Poly-Verbindungen aus Tris-bzw.Poly-(Oxobenzofuranyliden-Dihydroindolone) aus Tris- bzw.Poly-(Furanonverbindungen) oder Tris- bzw.Poly-(lsatin- oder Indoxylverbindungen) hergestellt werden. Die Herstellung von Polyisatinen ist aus GB-A 1,251,082 bekannt.

Selbstverständlich können viele Oxobenzofuranyliden-Dihydroindolone auch aus anderen Oxobenzofuranyliden-Dihydroindolonen hergestellt werden, indem deren Substituenten als funktionelle Gruppen ohne Veränderung des Oxobenzofuranyliden-Dihydroindolon-Grundgerüstes chemisch modifiziert werden. Der Fachmann kennt unzählige Methoden, womit Substituenten in andere Substituenten umgewandelt werden können, beispielsweise diejenigen, welche in der Serie "Compendium of Organic Synthetic Methods" (Wiley & Sons, New York, ab 1971) offenbart sind. Zweckmässige Reaktionsbedingungen sind solche, worunter aufgrund der bekannten Reaktivität des Oxobenzofuranyliden-Dihydroindolons nicht zu erwarten ist, dass dessen Lakton- oder Laktambindungen gespalten, oder dessen Doppelbindung reduziert oder sonst verändert wird. Zum Beispiel kann man neue Ester- oder Amid-Derivate nach allgemein bekannten Synthesemethoden herstellen wie sie zum Beispiel in Organic Syntheses, Collective Vol. I-VII, beschrieben werden. Bevorzugt werden insbesondere Ester die durch Umesterung oder Veresterung von Verbindungen der Formel (la), (Ib), (IIa) oder (IIb) beispielsweise mit diversen Alkoholen unter allgemein bekannten Synthese- und Katalyse-Bedingungen, wie zum Beispiel bei Temperaturen von 0°C bis 200°C, bei Alkoholmengen von 2 bis 200 Äquivalenten bezogen auf ein Äquivalent der Verbindung der Formeln (Ia), (Ib), (IIa) oder (IIb), gegebenenfalls in Gegenwart eines Lösungsmittels, hergestellt werden.

Verbindungen der Formeln (Ia), (Ib), (IIa) oder (IIb) werden zweckmässig in einer Menge von 0,01 bis 70 Gew.-%, üblicherweise von 0,01 bis 30 Gew.-%, vorzugsweise von 0,01 bis 10 Gew.-%, bezogen auf das zu färbende organische oder anorganische, hochmolekulare oder niedermolekulare, insbesondere hochmolekulare organische Material, eingesetzt.

Einen weiteren Erfindungsgegenstand bildet eine Zusammensetzung, enthaltend ein organisches oder anorganisches, hochmolekulares oder niedermolekulares, insbesondere hochmolekulares organisches Material, und mindestens eine erfindungsgemässe Verbindung oder die oben beschriebenen erfindungsgemässen Zusammensetzungen in einer färberisch wirksamen Menge, in der Regel im Bereich von 0,01 bis 70 Gew.-%, insbesondere von 0,01 bis 30 Gew.-%, bevorzugt von 0,01 bis 10 Gew.-%, bezogen auf das organische oder anorganische Material.

Ferner betrifft die vorliegende Erfindung die Verwendung der erfindungsgemässen Verbindungen oder Zusammensetzungen als Farbmittel, insbesondere zum Einfärben oder Pigmentieren von organischem oder anorganischem, hochmolekularem oder niedermolekularem, insbesondere hochmolekularem organischem Material.

Es ist aber ebenfalls möglich, die erfindungsgemässen Verbindungen und Zusammensetzungen als Gemische, feste Lösungen oder Mischkristalle, einzusetzen.

Die erfindungsgemässen Verbindungen können auch mit Farbmitteln einer anderen chemischen Klasse kombiniert werden, zum Beispiel mit Farbstoffen oder Pigmenten wie ausgewählt aus der Gruppe der Diketopyrrolopyrrole, Chinacridone, Perylene, Dioxazine, Anthrachinone, Indanthrone, Flavanthrone, Indigos, Thioindigos, Chinophthalone, Isoindolinone, Isoindoline, Phthalocyanine, Metallkomplexe, Azopigmente und Azofarbstoffe.

Je nach Art ihrer Substituenten und des zu färbenden Polymeren körmen die erfindungsgemässen Verbindungen als polymerlösliche Farbstoffe oder als Pigmente verwendet werden. Im letzteren Falle ist es vorteilhaft, die bei der Synthese anfallenden Produkte in eine feindisperse Form überzuführen. Dies kann auf an sich bekannte Weise geschehen. Je nach Verbindung und Verwendungszweck erweist es sich als vorteilhaft, die Farbmittel als Toner oder in Form von Präparaten zu verwenden.

Das hochmolekulare Material kann organisch oder anorganisch sein, und Kunststoffe und/oder Naturstoffe bedeuten. Es kann sich zum Beispiel um Naturharze oder trockene Öle, Kautschuk oder Casein oder um abgewandelte Naturstoffe wie Chlorkautschuk, ölmodifizierte Alkydharze, Viscose, um Celluloseether oder -ester wie Ethylcellulose, Celluloseacetat, -propionat, oder -butyrat, Celluloseactobutyrat sowie Nitrocellulose, handeln, insbesondere aber um vollsynthetische organische Polymere (Duro- und Thermoplaste), wie sie durch Polymerisation, zum Beispiel durch Polykondensation oder Polyaddition erhalten werden können. Zur Klasse der Polymeren gehören beispielsweise Polyolefine wie Polyethylen, Polypropylen, Polyisobutylen, ferner substituierte Polyolefine wie Polymerisate von Monomeren wie Vinylchlorid, Vinylacetat, Styrol, Acrylnitril, Acrylsäureester, Methacrylsäureester, Fluorpolymerisate wie zum Polyfluorethylen, Polytrifluorchlorethylen oder Tetrafluorethylen/Hexafluoropropylen-Mischpolymerisat sowie Copolymerisate der erwähnten Monomeren, insbesondere ABS (Acrylnitril/Butadien/Styrol) oder EVA (Ethylen/Vinylacetat). Aus der Reihe der Polyadditions- und Polykondensationsharze kann man beispielsweise Kondensationsprodukte von Formaldehyd mit Phenolen, die sogenannten Phenoplaste, und Kondensationsprodukte von Formaldehyd und Harnstoff oder Thioharnstoff, des weiteren Melamin, die sogenannten Aminoplaste, ferner die als Lackharze verwendeten Polyester, entweder gesättigte wie Alkydharze, als auch ungesättigte wie Maleinharze, ferner lineare Polyester, Polyamide, Polyurethane, Polycarbonate, Polyphenylenoxide, Silikone oder Silikonharze verwenden.

Die erwähnten hochmolekularen Verbindungen können einzeln oder in Gemischen als plastische Massen, Schmelzen oder in Form von Spinnlösungen vorliegen. Sie können auch in Form ihrer Monomeren oder im polymerisierten Zustand in gelöster Form als Filmbildner oder Bindemittel für Lacke oder Druckfarben vorliegen, wie Leinölfirnis, Nitrocellulose, Alkydharze, Melaminharze und Harnstoff-Formaldehydharze oder Acrylharze.

Die vorliegende Erfindung betrifft des weiteren die Verwendung der erfindungsgemässen Verbindungen oder Zusammensetzungen zur Herstellung von
Tinten, für Drucktinten in Druckverfahren, für den Flexodruck, Siebdruck, Verpackungsdruck, Sicherheitsfarbdruck, Tiefdruck oder Offsetdruck, für Druckvorstufen sowie für Textildruck, für Büro-, Heimanwendungen oder graphische Anwendungen wie beispielsweise für Papierwaren, für Kugelschreiber, Filzstifte, Faserstifte, Pappe, Holz, (Holz-)Beizen, Metall, Stempelkissen oder Tinten für Impact-printing Verfahren (mit Stossdruckfarbbänder); zur Herstellung von
Farbmitteln, für Lacke, die in der Industrie- oder im Gewerbe, für die Textildekoration und die industrielle Markierung genutzt werden können; insbesondere Farbmittel für Walz-, Pulver- oder Automobillacke, für high-solids (Iösungsmittelarme), für wasserhaltige oder metallische Lacke oder für pigmentierte Formulierungen, für wässerige Anstrichfarben, für Mineralöle, Schmierfette oder Wachse; zur Herstellung von
gefärbten Kunststoffen für Beschichtungen, Fasern, Platten oder Formträgern; zur Herstellung von
non-impact-printing Material (Nicht-Stossdruckmaterial) für digital-printing (Digitale Druckverfahren), für das thermische Wachs-Transfer-Druck-Verfahren, das Tintenstrahldruck-Verfahren oder für das thermische Transferdruck-Verfahren; zur Herstellung von
Farbfiltern, insbesondere für sichtbares Licht im Bereich von 400 bis 700 nm, für Flüssigkristallbildschirme (Liquid Crystal Display, LCD) oder Charge Combined Device (CCD); zur Herstellung von
Kosmetik oder zur Herstellung von
polymeren Farbpartikeln, von Tonern, dry copy toners (Trockenkopier-Farblacke), liquid copy toners (Flüssigkopier-Farblacke) oder elektrophotographische Toner.

Des weiteren betrifft die vorliegende Erfindung Tinten, enthaltend hochmolekulares organisches Material und eine färberisch wirksame Menge einer erfindungsgemässen Verbindung oder Zusammensetzung.

Verfahren zur Herstellung von Tinten insbesondere für den Tintenstrahldruck sind allgemein bekannt und zum Beispiel in US 5,106,417 beschrieben.

Man kann beispielsweise die Tinten herstellen, indem man die erfindungsgemässen Verbindungen mit polymeren Dispergierungsmitteln vermischt. Das Vermischen der erfindungsgemässen Verbindungen mit dem polymeren Dispergierungsmittel erfolgt bevorzugt nach allgemein bekannten Methoden des Vermischens wie Rühren oder Mixen, vorzugsweise empfiehlt es sich, Intensivmixer oder Hochleistungsrührer (wie Ultraturrax®) zu verwenden.

Beim Vermischen der erfindungsgemässen Verbindungen oder Zusammensetzungen mit polymeren Dispergierungsmitteln verwendet man zweckmässig ein mit Wasser verdünnbares organisches Lösungsmittel.

Zweckmässig wählt man das Gewichtsverhältnis der erfindungsgemässen Verbindungen oder Zusammensetzungen zur Tinte im Bereich von 0,0001 bis 75 Gew.-%, bevorzugt von 0,001 bis 50 Gew.-%, bezogen auf das Gesamtgewicht der Tinte.

Die vorliegende Erfindung betrifft auch ein Verfahren zur Herstellung von Tinten, indem man hochmolekulares organisches Material mit einer färberisch wirksamen Menge der erfindungsgemässen Verbindungen oder Zusammensetzungen miteinander vermischt.

Ferner betrifft die vorliegende Erfindung Farbmittel enthaltend hochmolekulares organisches Material und eine erfindungsgemässe Verbindung oder Zusammensetzung in einer färberisch wirksamen Menge.

Zudem betrifft die vorliegende Erfindung ein Verfahren zur Herstellung von Farbmittel, indem man ein hochmolekulares organisches Material und eine färberisch wirksame Menge einer erfindungsgemässen Verbindung oder Zusammensetzung vermischt.

Des weiteren betrifft die vorliegende Erfindung gefärbte Kunststoffe oder polymere Farbpartikel enthaltend ein hochmolekulares organisches Material und eine erfindungsgemässe Verbindung oder Zusammensetzung in einer färberisch wirksamen Menge.

Zudem betrifft die vorliegende Erfindung ein Verfahren zur Herstellung gefärbter Kunststoffe oder polymerer Farbpartikel, indem man ein hochmolekulares organisches Material und eine färberisch wirksame Menge einer erfindungsgemässen Verbindung oder Zusammensetzung miteinander vermischt.

Die Einfärbung der hochmolekularen, organischen Substanzen mit den erfindungsgemässen Farbmitteln oder Zusammensetzungen erfolgt beispielsweise derart, dass man ein solches Farbmittel gegebenenfalls in Form von Masterbatches, diesen Substraten unter Verwendung von Walzwerken, Misch- oder Mahlapparaten zumischt, wodurch das Farbmittel im hochmolekularen Material gelöst oder fein verteilt wird. Das hochmolekulare organische Material mit dem beigemischten Farbmittel wird in der Regel hierauf nach an sich bekannten Verfahren verarbeitet wie Kalandrieren, Pressen, Strangpressen, Streichen, Spinnen, Giessen oder durch Spritzguss, wodurch das eingefärbte Material üblicherweise seine endgültige Form bekommt. Das Beimischen des Farbmittels kann auch unmittelbar vor dem eigentlichen Verarbeitungsschritt durchgeführt werden, indem beispielsweise ein pulverförmiges erfindungsgemässes Farbmittel und ein granuliertes hochmolekulares organisches Material, sowie gegebenenfalls auch Zusatzstoffe wie beispielsweise Additive, gleichzeitig direkt der Einlasszone einer Strangpresse kontinuierlich zudosiert werden, wo das Einmischen noch knapp vor der Verarbeitung stattfindet. Im allgemeinen ist jedoch ein vorgängiges Einmischen des Farbmittels ins hochmolekulare organische Material bevorzugt, da gleichmässigere Resultate erhalten werden können.
Oft ist es erwünscht, zur Herstellung von nicht starren Formlingen oder zur Verringerung ihrer Sprödigkeit den hochmolekularen Verbindungen vor der Verformung sogenannte Weichmacher zuzusetzen. Als solche können zum Beispiel Ester der Phosphorsäure, Phthalsäure oder Sebacinsäure dienen. Die Weichmacher können im erfindungsgemässen Verfahren vor oder nach der Einverleibung des Farbmittels in die Polymeren eingearbeitet werden. Es ist ferner möglich, zwecks Erzielung verschiedener Farbtöne den hochmolekularen, organischen Stoffen neben den erfindungsgemässen Verbindungen oder Zusammensetzungen Bestandteile wie Weiss-, Bunt- oder Schwarzpigmente in den jeweils gewünschten Mengen zuzufügen.

Zum Einfärben von Lacken und Druckfarben werden die hochmolekularen organischen Materialien und die erfindungsgemässen Verbindungen oder Zusammensetzungen, gegebenenfalls zusammen mit Zusatzstoffen wie Füllmitteln, Farbstoffen, Pigmenten, Siccativen oder Weichmachern, in einem gemeinsamen organischen Lösungsmittel oder Lösungsmittelgemisch fein dispergiert oder gelöst. Man kann dabei so verfahren, dass man die einzelnen Komponenten für sich oder auch mehrere gemeinsam dispergiert oder löst, und erst hierauf alle Komponenten zusammenbringt. Die Verarbeitung erfolgt nach üblichen Methoden, beispielsweise durch Spritzen, Filmziehen oder einer der vielen Druckmethoden, worauf der Lack oder die Druckfarbe, gegebenenfalls nach vorheriger Trocknung, zweckmässig thermisch oder durch Bestrahlung gehärtet wird.

Handelt es sich beim zu färbenden hochmolekularen Material um einen Lack, so kann es sich um eine gewöhnlichen Lack, oder auch um einen Speziallack, beispieksweise einen Automobillack, bevorzugt um eine beispielsweise Metall- oder Glimmer-Partikel enthaltende Metalleffektlackierung, handeln.

Bevorzugt ist die Einfärbung von thermoplastischen Kunststoffen, insbesondere auch in Form von Fasern sowie von Druckfarben. Bevorzugte, erfindungsgemäss einfärbbare hochmolekulare organische Materialien sind ganz allgemein Polymere mit einer Dielektrizitätskonstante ≥2,5, insbesondere Polyester, Polycarbonat (PC), Polystyrol (PS), Polymethylmethacrylat (PMMA), Polyamid, Polyethylen, Polypropylen, Styrol/Acrylnitril (SAN) oder Acrylnitril/Butadien/Styrol (ABS). Besonders bevorzugt sind Polyester, Polycarbonat, Polystyrol und PMMA. Ganz besonders bevorzugt sind Polyester, Polycarbonat oder PMMA, insbesondere aromatische Polyester, welche durch Polykondensation von Terephthalsäure erhalten werden können, wie beispielsweise Polyethylenterephthalat (PET) oder Polybutylenterephthalat (PBTP).

Des weiteren besonders bevorzugt ist die Einfärbung von Mineralölen, Schmierfetten und Wachsen mit den erfindungsgemässen Verbindungen.

Ausserdem betrifft die vorliegende Erfindung Non-impact-printing-Material, enthaltend hochmolekulares organisches Material und eine erfindungsgemässe Verbindung oder Zusammensetzung in einer färberisch wirksamen Menge.

Zudem betrifft die vorliegende Erfindung ein Verfahren zur Herstellung von Non-impact-printing-Material, indem man ein hochmolekulares organisches Material und eine färberisch wirksame Menge einer erfindungsgemässen Verbindung oder Zusammensetzung miteinander vermischt.

Ferner betrifft die vorliegende Erfindung ein Verfahren zur Herstellung von Farbfiltern enthaltend ein transparentes Substrat sowie darauf aufgebracht eine rote, blaue und grüne Schicht in beliebiger Reihenfolge, indem man zur Herstellung der roten, blauen und grünen Schichten je eine entsprechend gefärbte erfindungsgemässe Verbindung oder Zusammensetzung verwendet.

Die verschiedenfarbigen Schichten weisen bevorzugt derartige Muster auf, dass sie auf mindestens 5% ihrer jeweiligen Fläche nicht überlappen und ganz besonders bevorzugt überhaupt nicht überlappen.

Die Farbfilter lassen sich beispielsweise unter Verwendung von Tinten, insbesondere Drucktinten, die die erfindungsgemässen Verbindungen oder Zusammensetzungen enthalten, beschichten, oder beispielsweise durch Vermischung einer erfindungsgemässen Verbindungen oder Zusammensetzungen mit chemisch, thermisch oder photolytisch strukturierbarem hochmolekularem Material ("resist") herstellen. Die weitere Herstellung kann man beispielsweise analog zu der in EP-A 654 711 beschriebenen Methode durch Aufbringen auf ein Substrat, wie ein LCD, anschliessende Photostrukturierung und Entwickeln durchführen.

Des weiteren umfasst die Erfindung ein transparentes Substrat, das mit je einer roten, blauen und grünen Schicht je einer entsprechend gefärbten erfindungsgemässen
Verbindung oder Zusammensetzung, enthaltend pigmentiertes hochmolekulares organisches Material, beschichtet ist. Die Reihenfolge der Beschichtung spielt in der Regel keine Rolle. Bevorzugt weisen die verschiedenfarbigen Schichten derartige Muster auf, dass sie auf mindestens 5% ihrer jeweiligen Fläche nicht überlappen, besonders bevorzugt überhaupt nicht überlappen.

Ferner umfasst die vorliegende Erfindung auch Farbfilter enthaltend ein transparentes Substrat sowie darauf aufgebracht eine rote, blaue und grüne Schicht, je erhältlich aus einer entsprechend gefärbten erfindungsgemässen Verbindung oder Zusammensetzung.

Zudem betrifft die vorliegende Erfindung Toner enthaltend hochmolekulares organisches Material und eine erfindungsgemässe oder Zusammensetzung in einer färberisch wirksamen Menge.

Ausserdem betrifft die vorliegende Erfindung Verfahren zur Herstellung von Tonern, indem man ein hochmolekulares organisches Material und eine färberisch wirksame Menge einer erfindungsgemässen oder erfindungsgemäss hergestellten Verbindung oder Zusammensetzung miteinander vermischt.

In einer besonderen Ausführungsform des erfindungsgemässen Verfahrens werden Toner, Lacke, Tinten oder gefärbte Kunststoffe hergestellt, indem man Masterbatches von Tonern, Lacken, Tinten oder gefärbten Kunststoffen in Walzwerken, Misch- oder Mahlapparaten bearbeitet.
Eine färberisch wirksame Menge einer erfindungsgemässen oder erfindungsgemäss hergestellten Verbindung oder Zusammensetzung bedeutet in der vorliegenden Erfindung in der Regel 0,0001 bis 99,99 Gew.-%, bevorzugt 0,001 bis 50 Gew.-% und besonders bevorzugt 0,01 bis 50 Gew.-%, bezogen auf das Gesamtgewicht des damit gefärbten oder pigmentierten Materials.

Wenn die erfindungsgemässen oder erfindungsgemäss hergestellten Verbindungen oder Zusammensetzungen in den angewandten Polymeren gelöst vorliegen, zeichnen sie sich durch einen reinen Farbton, grosse Farbstärke, hohe Licht- und Wetterechtheit sowie Hitzeechtheit, insbesondere von PET, PMMA, PS und PC und ausserdem durch hohe Fluoreszenz aus. Die erhaltenen Färbungen, beispielsweise in thermoplastischen oder duroplastischen Kunststoffen, Fasern, Lacken oder Druckfarben, zeichnen sich durch einen reinen Farbton, hohe Farbstärke, hohe Sättigung, hohe Transparenz, gute Überlackier-, Migrations-, Reib-, Licht-, Wetter- und insbesondere Hitzeechtheit sowie durch einen guten Glanz aus. Die Farbmittel besitzen eine gute Dispergierbarkeit und im allgemeinen gute Löslichkeiten in organischen Lösungsmitteln. Sie eignen sich in Sonnenenergie-Kollektoren und zur Herstellung von Laser-Strahlen. In Mischungen enthaltend die erfindungsgemässen Verbindungen, werden wunderschöne Farbnuancen erhalten. Besonders vorteilhaft ermöglichen asymmetrische Oxobenzofuranyliden-Dihydroindolone sowie Bis- oder Tris-(Oxobenzofuranyliden-Dihydroindolone) weitere Farbtöne und bieten die Möglichkeit, durch die Wahl der Substituenten, deren Löslichkeit zu beeinflussen.

### Beispiele

### Beispiel 1: 3-(5,7-Di-tert-butyl-2-oxo-benzofuran-3-yliden)-1,3-dihydro-indol-2-on:

14,7 g (0,1 Mol) Isatin, 24,6 g (0,1 Mol) 5,7-Di-tert-butyl-3H-benzofuran-2-on, hergestellt analog der Methode von H.-D. Becker und K. Gustaffson, die in J. Org. Chem., 42, 2966 von 1977 beschrieben ist, und 0,75 g p-Toluolsulfonsäure in 75 ml Essigsäure werden 17 h unter Stickstoff zum Rückfluss erhitzt. Das Reaktionsgemisch wird auf Raumtemperatur abgekühlt, der Niederschlag wird abfiltriert, und der Filterrückstand mit Methanol gewaschen und anschliessend bei 60°C getrocknet. Die Ausbeute beträgt 27,7 g
(74% d. Th.). Schmelzpunkt: 277-284°C.
C: ber./gef. 76,77/76,75; H: ber./gef. 6,71/6,74; N: ber./gef. 3,73/3,56

### Beispiel 2: 3-[7-tert-Butyl-2-oxo-3-(2-oxo-1,2-dihydro-indol-3-yliden)-2,3-dihydro-benzofuran-5-yl]-propionsäure:

A) 3-(7-tert-Butyl-2-oxo-2,3-dihydro-benzofuran-5-yl)-propionsäure
   66,7 g (0,3 Mol) 3-(3-tert-Butyl-4-hydroxy-phenyl)-propionsäure (hergestellt analog zu der in JP-A2- 63/227542 beschriebenen Methode), in 58 g (0,4 Mol) 40 gew.-%iger, wässriger Glyoxal-Lösung und 3 ml 32 gew.-%iger Salzsäure werden in 200 ml Essigsäure 6 h zum Rückfluss erhitzt. Danach wird das Gemisch in 600 ml Eiswasser gegossen, und der Niederschlag abfiltriert. Der Filterrückstand wird mit Wasser gewaschen und anschliessend bei 60°C g getrocknet. Durch Umkristallisieren aus Toluol werden 54,3 g (69% d.Th.) 3-(7-tert-Butyl-2-oxo-2,3-dihydro-benzofuran-5-yl)-propionsäure erhalten. Schmp. 167-169°C
B) 7,36 g (0,05 Mol) Isatin, 13,1 g (0,05 Mol) 3-(7-tert-Butyl-2-oxo-2,3-dihydro-benzofuran-5-yl)-propionsäure aus Beispiel 2A und 0,5 g p-Toluolsulfonsäure werden unter Stickstoff in 50 ml Essigsäure 23 h zum Rückfluss erhitzt. Das Reaktionsgemisch wird auf Raumtemperatur abgekühlt, der ausgefallene Feststoff wird abfiltriert, und der Filterrückstand mit Wasser gewaschen und bei 60°C getrocknet. Die Ausbeute beträgt
   12,2 g (63% d.Th.). Schmp. 228-252°C.
C: ber./gef. 70,58/70,31; H: ber./gef. 5,41/5,52; N: ber./gef. 3,58/3,40

### Beispiel 3: 3-(5,7-Di-tert-butyl-2-oxo-benzofuran-3-ylidene)-1-methyl-1,3-dihydro-indol-2-on

8,05 g (0,05 Mol) N-Methylisatin (von Aldrich), 12,3 g (0,05 Mol) 5,7-Di-tert-butyl-3Hbenzofuran-2-on und 0,4 g p-Toluolsulfonsäure werden unter Stickstoff in 40 ml Essigsäure 19 h zum Rückfluss erhitzt. Die Suspension wird auf Raumtemperatur abgekühlt, der ausgefallene Feststoff wird abfiltriert und der Filterrückstand mit Methanol gewaschen und anschliessend bei 60°C getrocknet. Die Ausbeute beträgt 15,3 g (79% d.Th.).
Schmp.: 235-237°C.
C: ber./gef. 77,09/77,06; H: ber./gef. 6,99/7,07; N: ber./gef. 3,60/3,88

### Beispiel 4: 3-[7-tert-Butyl-3-(1-methyl-2-oxo-1,2-dihydro-indol-3-yliden)-2-oxo-2,3-dihydrobenzofuran-5-yl]-propionsäure:

16,1 g (0,1 Mol) N-Methylisatin, 26,2 g (0,1 Mol) 3-(7-tert-Butyl-2-oxo-2,3-dihydrobenzofuran-5-yl)-propionsäure (siehe Beispiel 2) und 2 g p-Toluolsulfonsäure werden unter Stickstoff in 80 ml Essigsäure 24 h zum Rückfluss erhitzt. Die Suspension wird auf Raumtemperatur abgekühlt und mit 80 ml Methyl-tert.-butyl-ether verdünnt und anschliessend filtriert. Der Filterrückstand wird mit Methyl-tett.-butyl-ether gewaschen und anschliessend bei 60°C getrocknet. Die Ausbeute beträgt 28,5 g (70% d.Th.).
Schmp.: 231-232°C.
C: ber./gef. 71,10/71,03; H: ber./gef. 5,72/5,62; N: ber./gef. 3,45/3,62

### Beispiel 5: 3-(7-tert-Butyl-5-methoxy-2-oxo-benzofuran-3-yliden)-1,3-dihydro-indol-2-on (Z/E-Gemisch.

6,6 g (0,03 Mol) 7-tert.-Butyl-5-methoxy-3H-benzofuran-2-on (hergestellt analog zu der Methode aus J. Org. Chem., 42 (1977) 2966) (man erhält farblose Kristalle mit einem Schmp. 116-118°C), 4,4 g (0,03 Mol) Isatin und 0,25 g p-Toluolsulfonsäure werden unter Stickstoff in 25 ml Essigsäure 15 h zum Rückfluss erhitzt. Danach werden 50 ml Ethanol zugegeben und die Suspension auf Raumtemperatur abgekühlt und filtriert. Der Filterrückstand wird mit Methanol gewaschen und anschliessend bei 60°C getrocknet. Die Ausbeute beträgt 4,9 g (47% d.Th). Schmp.: 247-258°C. C: ber./gef. 72,19/72,09;
H: ber./gef. 5,48/5,52; N: ber./gef. 4,01/4,09

### Beispiel 6: 3-(7-tert-Butyl-5-methoxy-2-oxo-benzofuran-3-yliden)-1-methyl-1,3-dihydro-indol-2-on:

11,0 g (0,05 Mol) 7-tert-Butyl-5-methoxy-3H-benzofuran-2-on und 8,1 g (0,05 Mol) N-Methylisatin und 1 g p-Toluolsulfonsäure werden unter Stickstoff in 40 ml Essigsäure 24 h zum Sieden erhitzt. Danach werden 40 ml Methanol zugegeben und die Suspension auf Raumtemperatur abgekühlt und abfiltriert. Der Filterrückstand wird mit Methanol gewaschen und anschliessend mit 100 ml n-Butanol versehen und 15 Minuten zum Rückfluss erhitzt. Anschliessend wird auf 5°C abgekühlt und abfiltriert. Der Filterrückstand wird anschliessend bei 60°C getrocknet. Man erhält 13,4 g (74% d.Th.).
Schmp.: 188-190°C.
C: ber./gef. 72,71/72,52; H: ber./gef. 5,82/5,69; N: ber./gef. 3,85/3,65

### Beispiel 7: [3-(7-tert-Butyl-5-methoxy-2-oxo-benzofuran-3-yliden)-2-oxo-2,3-dihydro-indol-1-yl]-essigsäure:

A) (2,3-Dioxo-2,3-dihydro-indol-1-yl)-essigsäure-tert.butyl ester:
   44,1 g (0,3 Mol) Isatin, 62,5 g (0,45 Mol) Kaliumcarbonat und 66,2 g(0,34 Mol) Bromessigsäure-tert.butyl-ester (von Aldrich) werden in 100 ml N,N'-Dimethylformamid bei Raumtemperatur 24 h gerührt. Das Gemisch wird dann in 800 ml Wasser gegossen, der ausgefallene Niederschlag abfiltriert, mit Wasser gewaschen und bei 60°C getrocknet. Nach einmaligem Umkristallisieren aus Toluol - Hexan (1:1) werden 608 g (78% d.Th.) Produkt mit einem Schmp. von 119-121°C erhalten.
B) 25,4 g (0,097 Mol) des oben erhaltenen (2,3-Dioxo-2,3-dihydro-indol-1-yl)-essigsäuretert.-butylesters und 1 g p-Toluolsulfonsäure werden unter Stickstoff in 50 ml Essigsäure 16 h zum Sieden erhitzt. Danach werden 50 ml Wasser zugegeben und die erhaltene Suspension wird daraufhin auf Raumtemperatur abgekühlt. Der Feststoff wird abfiltriert, und der Filterrückstand mit 50 gew.-%iger Essigsäure gewaschen und bei
   60°C getrocknet. Die Ausbeute beträgt 31,8 g (80% d. Th.). Schmp.: 264-266°C;
C: ber./gef. 67,81/67,79; H: ber./gef. 5,20/4,94; N: ber./gef. 3,44/3,32

### Beispiel 8: 5,7-Dibromo-3-(5,7-di-tert-butyl-2-oxo-benzofuran-3-yliden)-1,3-dihydro-indol-2-on (Z/E-Gemisch):

7,6 g (0,025 Mol) 5,7-Dibromisatin (hergestellt analog zu der in Collect. Czech. Chem. Commun., 55 (1990) 2963 beschriebenen Methode), 5,5 g (0,025 Mol) 7-tert.-Butyl-5-methoxy-3H-benzofuran-2-on und 0,25 g p-Toluolsulfonsäure werden unter Stickstoff in 25 ml Essigsäure 17 h zum Sieden erhitzt. Danach werden 40 ml Methanol zugegeben und die erhaltene Suspension wird auf 5°C abgekühlt. Der ausgefallene Feststoff wird abfiltriert, der Filterrückstand mit Methanol gewaschen und bei 60°C getrocknet. Die Ausbeute beträgt 11,9 g (94% d.Th.). Schmp.: 232-256°C.
C: ber./gef. 54,06/54,35; H: ber./gef. 4,35/4,22; N: ber./gef. 2,63/2,57

### Beispiel 9: 5,7-Dibromo-3-(7-.tert.-butyl-5-methoxy-2-oxo-benzofuran-3-yliden)-1,3-dihydroindol-2-on (Z/E-Gemisch)

7,6 g (0,025 Mol) 5,7-Dibromisatin, 6,15 g (0,025 Mol) 5,7-Di-tert.-butyl-3H-benzofuran-2-on und 0,5 g p-Toluolsulfonsäure werden unter Stickstoff in 25 ml Essigsäure 21 h zum Sieden erhitzt. Danach werden 40 ml Methanol zugegeben und die so erhaltene Suspension wird auf 5°C gekühlt. Der daraufhin erhaltene Feststoff wird abfiltriert, der Filterrückstand mit Methanol gewaschen und bei 60°C getrocknet. Die Ausbeute beträgt 10,7 g (80% d.Th.). Schmp.: 303-314°C. C: ber./gef. 49,73/49,46; H: ber./gef. 3,38/3,45; N: ber./gef. 2,76/2,73

### Beispiel 10: 2-(5,7-Di-tert-butyl-2-oxo-benzofuran-3-yliden)-1,2-dihydro-indol-3-on:

20 g (0,08 Mol) 5,7-Di-tert-butyl-3H-benzofuran-2-on, 13,2 g (0,08 Mol) 2-Chloro-indol-3-on (hergestellt analog zu der in Synthesis, 496 (1974) beschriebenen Methode) in 150 ml Toluol und 30 ml Triethylamin werden 4 h bei 40°C gerührt. Danach werden 500 ml Ethylacetat zugegeben und die so erhaltene Mischung wird mit 500 ml 5 gew.-%iger verdünnter Salzsäure und danach mit 500 ml Wasser gewaschen. Die abgetrennte Ethylacetat-Phase wird anschliessend über Natriumsulfat getrocknet. Der so erhaltene Feststoff wird nach Abdestillation des Lösungsmittels aus Butanol-Toluol (1:1) umkristallisiert. Die Ausbeute beträgt 9,3 g (31% d.Th.).
Schmp.: 290-292°C.
C: ber/gef. 76,77/76,94; H: ber./gef. 6,71/6,89; N: ber./gef. 3,73/3,61

### Beispiel 11: 2-(7-tert-Butyl-5-methoxy-2-oxo-benzofuran-3-yliden)-1,2-dihydro-indol-3-on:

23,5 g (0,160 Mol) Isatin werden in 200 ml Chlorbenzol vorgelegt und mit 35 g (0,168 Mol) Phosphorpentachlorid versetzt. Die Suspension wird langsam unter lebhafter HCl-Bildung auf 100°C erhitzt und bei dieser Temperatur 90 Minuten gerührt. Danach werden 100 ml Chlorbenzol und gebildetes Phosphoroxychlorid abdestilliert. Die zurückbleibende Lösung wird mit 35,2 g (0,160 Mol) 7-tert.-Butyl-5-methoxy-3H-benzofuran-2-on versetzt, anschliessend 2 h bei 130°C nachgerührt und dann durch Abdestillieren von 50 ml Chlorbenzol aufkonzentriert. Anschliessend werden 200 ml Methanol zugegeben, dann die Suspension auf 5°C abgekühlt und danach filtriert. Der Filterrückstand wird durch Chromatographie auf Kieselgel mit einem Lösungsmittelgemisch von Toluol, Ethylacetat und Methanol im Mischungsverhältnis (4:3:3) gereinigt. Die reinen Fraktionen werden einmal aus Butanol-Toluol (1:1) umkristallisiert und bei 60°C getrocknet. Man erhält eine Ausbeute von 6,7 g (12% d. Th.).
Schmp.: 239-240°C.
C: ber./gef. 72,19/72,03; H: ber./gef. 5,48/5,42; N: ber./gef. 4,01/4,17

### Beispiel 12a: Herstellung des Bisbenzofuranons

2,5-Dihydroxybenzol-1,4-diessigsäure, 10 g (44,2 mmol), (käuflich bei Aldrich) Toluol, 500 ml, und Essigsäureanhydrid, 100 ml, werden bei 90°C 4,5 h lang umgesetzt. Anschliessend wird das Reaktionsgemisch auf 25°C abgekühlt und filtriert. Der Filterrückstand wird mit Toluol, 50 ml, gewaschen und dann im Vakuumtrockenschrank bei 50°C getrocknet. Man erhält 7,1 g des Bisbenzofuranons

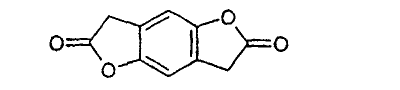

Die Ausbeute beträgt 85% d. Th..

### Beispiel 12b:Umsetzung des Bisbenzofuranons mit Isatin

Bisbenzofuranons, 2,0 g (hergestellt nach Beispiel 12a), Isatin, 3,09 g (käuflich bei Fluka purum) und Toluonsulfonsäure, 0,5 g, (käuflich bei Fluka purum) werden in Essigsäure, 30 ml, bei 115°C 16 h lang gerührt. Das Reaktionsgemisch wird abfiliriert und der Filterrückstand mit Essigsäure, 30 ml, und Methanol, 50 ml, gewaschen, und anschliessend im Vakuumtrockenschrank bei 80°C getrocknet. Man erhält 4,5 g eines violetten Pulvers der Verbindung untenstehender Formel 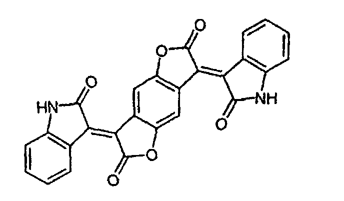 LDI-TOF(laser desorption ionisation/time of light): (M⁻) = 448

### Beispiel 13: Herstellung von Spritzgussplatten

Allgemeine Vorschrift: Ein erfindungsgemässes Oxobenzofuranyliden-Dihydroindolon wird mit 1500 g bei 473-543 K vorgetrocknetem Kunststoff kurz von Hand dann während 5 min bei 50 U/min auf einem Taumelmischer gemischt. Diese Mischung wird anschliessend auf einem 25 mm 1 Schnecken-Extruder (Collin) bei 543 K vorextrudiert. Anschliessend wird das Extrudat auf einer Mikroprozessor-gesteuerten Spritzgussmaschine (™Ferromatik FM 40, Klöckner) zu Spritzlingen (65 mm × 25 mm × 1,5 mm grosse Plättchen) verarbeitet. Die Verweilzeit des Polymers beträgt, wenn nicht anders vermerkt, 5 Minuten, wobei der Staudruck und die Schneckendrehzahl niedrig gehalten werden.
(a) 0,1 g des in Beispiel 1 hergestellten Oxobenzofuranyliden-Dihydroindolons wird mit Polyethylenterephthalat ("PET") (™MELINAR PURA, ICI) entsprechend der allgemeinen Vorschrift zu Spritzlingen verarbeitet. Dabei werden die Spritzlinge jeweils bei (a1) 543 K, (a2) 553, (a3) 563 K und (a4) 573 K in der Spritzgussmaschine hergestellt.
   Das Beispiel wird mit den Oxobenzofuranyliden-Dihydroindolonen der Beispiele 2 bis 11 wiederholt.
   Die Spritzlinge zeichnen sich durch ausgezeichnete Farbechtheiten, sehr hohe Hitzestabilität, hohe Lichtechtheit, gute Migrationsbeständigkeit und hohe Farbstärke aus. (Die Farbechtheiten werden visuell gegenüber einem Standard oder farbmetrisch bestimmt.)
(b) Beispiel 13a wird wiederholt mit dem Unterschied, dass man als Kunststoff ein Polybutylenterephthalat ("PBTB") (CRASTIN®S 600 von Ciba Specialty Chemicals) einsetzt. Des weiteren werden die Spritzlinge (verschiedene Batches durch Wahl unterschiedlicher Temperaturen) jeweils während 2 Minuten Verweilzeit bei (b1) 533 K, (b2) 548 K und (b3) 563 K hergestellt.

Das Beispiel wird mit den Oxobenzofuranyliden-Dihydroindolonen der Beispiele 2 bis 12 wiederholt.

Die Spritzlinge zeichnen sich durch ausgezeichnete Farbechtheiten, sehr hohe Hitzestabilität, hohe Lichtechtheit, gute Migrationsbeständigkeit und hohe Farbstärke aus. (Die Farbechtheiten werden visuell gegenüber einem Standard oder farbmetrisch bestimmt.)

## Patentansprüche

1. Oxobenzofuranyliden-Dihydroindolone der Formeln trans-(Ia) und cis-(Ib) 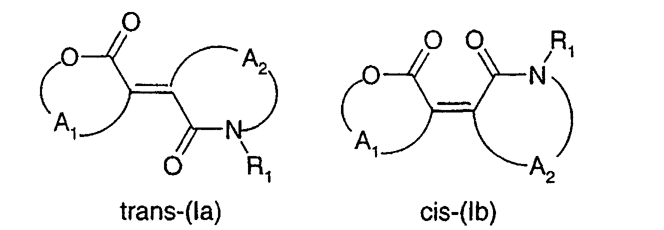 sowie der Formeln trans-(IIa) und cis-(IIb) 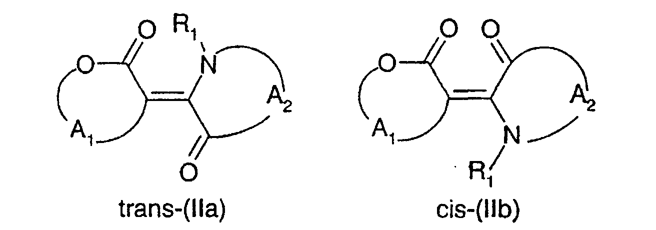 worin
A₁ und A₂ unabhängig voneinander unsubstituiertes oder 1- bis 4-fach substituiertes ortho-C₆-C₁₈-Aryien bedeuten, und
R₁ für Wasserstoff, C₁-C₂₅-Alkyl, C₅-C₁₂-Cycloalkyl, C₆-C₂₄-Aryl, einen heteroaromatischen Rest, -(CH₂)ₙCOR₂ oder -(CH₂)ₘ-OR₃, steht,
worin R₂ für Hydroxy, -OX, unsubstituiertes oder ein- oder mehrfach mit Hydroxy, -OX, oder Amino substituiertes C₁-C₂₅-Alkoxy, C₁-C₂₅-Alkylamino oder C₁-C₂₅-Dialkylamino, Di-(C₆-C₂₄-Aryl)-amino, C₁-C₁₂-Alkyl, C₂-C₂₄-Alkenyl steht, und
X ein Kation bedeutet, und
R₃ für Wasserstoff oder-CO-(C₁-C₂₅-Alkyl) steht, und
n und m unabhängig voneinander für eine ganze Zahl im Bereich von 0 bis 6 stehen, und
eine C-C-Einfachbindung auch durch eine entsprechende Ethereinheit, C-O-C, ersetzt sein kann,
mit der Massgabe, dass, wenn R₁ für Wasserstoff und A₂ für 1,2-Phenylen stehen, A₁ nicht für 9,10-Anthrachinon-1,2-ylen, 4-Chlor-3,5-dimethyl-1,2-phenylen oder 3,5-Dimethyl-1,2-phenylen steht.

2. Oxobenzofuranyliden-Dihydroindolone gemäss Anspruch 1, **dadurch gekennzeichnet, dass** A₁ und/oder A₂ für substituiertes oder unsubstituiertes 1,2-Phenylen, 1,2-Naphthylen, 2,3-Naphthylen, 1,2-Phenanthrylen, 2,3-Phenanthrylen, 3,4-Phenanthrylen, 9,10-Phenanthrylen, 1,2-Anthracenyl, 2,3-Anthracenyl oder 1,2- Anthrachinonylen oder 2,3-Anthrachinonylen steht.

3. Oxobenzofuranyliden-Dihydroindolone gemäss einem der Ansprüche 1 oder 2, **dadurch gekennzeichnet, dass** A₁ für 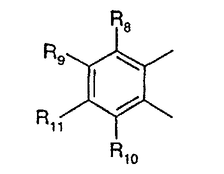 und A₂ für 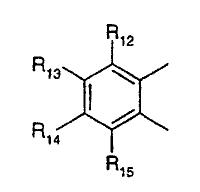 stehen, worin
R₈, R₉, R₁₁, R₁₀, R₁₂, R₁₃, R₁₄ und R₁₅ unabhängig voneinander Wasserstoff, Halogen, Cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂-Alkylen)-COOX, -O-(C₁-C₁₂-Alkylen)-COOH, -O-(C₁-C₁₂-Alkylen)-O-CO-R₁₇, -O-(C₁-C₁₂-Alkylen)-COOR₁₇, -O-(C₁-C₁₂-Alkylen)-CONR₁₉R₂₀, -O-(C₁-C₁₂-Alkylen)-OR₁₇, -(C₁-C₁₂-Alkylen)-O-CO-R₁₇, -(C₁-C₁₂-Alkylen)-OR₁₇, -COOR₁₇, -(C₁-C₁₂-Alkylen)-COOR₁₇, -(C₁-C₁₂-Alkylen)-CONR₁₉R₂₀, -(C₁-C₁₂-Alkylen)-COOX, -(C₁-C₁₂-Alkylen)-COOH, -COOX,-COOH, -SR₁₇, -SOR₁₇, -SO₂R₁₇, -SO₂NR₁₉R₂₀, -SO₃R₁₇, SO₃H oder SO₃X bedeuten,
worin
R₁₇, R₁₈, R₁₉ und R₂₀ unabhängig voneinander für Wasserstoff oder R₁₆ stehen, und
R₁₆ für unsubstituiertes oder einfach oder mehrfach mit Halogen, Hydroxy, Amino, Oxo, Carboxy, Cyano, -COOR₁₈ oder -COOX substituiertes C₁-C₂₅Alkyl, C₅-C₁₂-Cycloalkyl oder C₂-C₂₄Alkenyl, welches ununterbrochen oder einfach oder mehrfach durch O, S oder N-( C₁-C₂₅Alkyl), N-(C₂-C₂₄Alkenyl) unterbrochen sein kann, falls das Alkyl mehr als zwei und das Alkenyl mehr als drei Kohlenstoffatome aufweist, oder für unsubstituiertes oder einfach oder mehrfach mit Halogen, Nitro, Cyano, -OR₁₈, -SR₁₈,
-NR₁₉R₂₀, -CONR₁₉R₂₀, -COOR₁₈, -COOX, -COOH, -SO₂R₁₈, -SO₂NR₁₉R₂₀,-SO₃R₁₈, -SO₃X, -SO₃H, -NR₁₉COR₁₈ oder -NR₁₉COOR₁₈ substituiertes C₆-C₁₈Aryl, C₇-C₁₈Aralkyl oder Heteroaryl steht,
oder
R₁₉ und R₂₀ zusammen mit dem sie verbindenden Stickstoffatom für unsubstituiertes oder mit C₁-C₄Alkyl einfach bis vierfach substituiertes Pyrrolidinyl, Piperidinyl, Piperazinyl oder Morpholinyl, oder für Carbazolyl, Phenoxazinyl oder Phenothiazinyl stehen,
wobei gegebenenfalls jeweils R₈ und R₉, R₉ und R₁₁, R₁₁ und R₁₀ sowie R₁₂ und R₁₃, R₁₃ und R₁₄ oder R₁₄ und R₁₅ zusätzlich einen substituierten oder unsubstituierten 5- oder 6-gliedrigen Ring bilden können.

4. Oxobenzofuranyliden-Dihydroindolone gemäss Anspruch 3, **dadurch gekennzeichnet, dass** mindestens einer der Reste R₈, R₉, R₁₁, R₁₀, R₁₂, R₁₃, R₁₄ und R₁₅ für einen Rest, ausgewählt aus der Gruppe, bestehend aus den Resten Z₁, Z₂, Z₃ und Z₄ steht 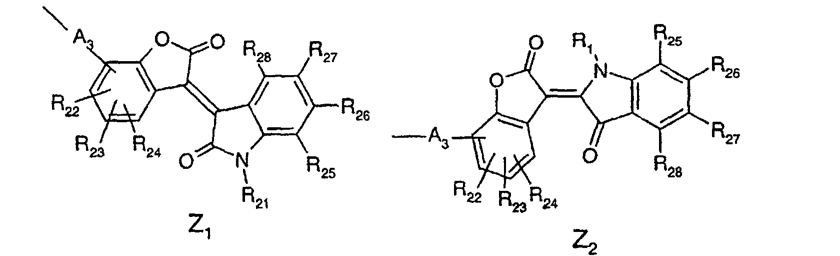 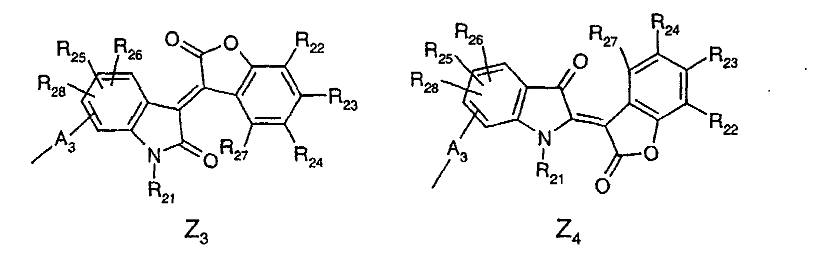 worin
R₂₂, R₂₃, R₂₄, R₂₅, R₂₆, R₂₇ und R₂₈ unabhängig voneinander für Wasserstoff, Halogen, Cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂-Alkylen)-COOX, -O-(C₁-C₁₂-Alkylen)-COOH, -O-(C₁-C₁₂-Alkylen)-O-CO-R₁₇, -O-(C₁-C₁₂-Alkylen)-COOR₁₇, -O-(C₁-C₁₂-Alkylen)-CONR₁₉R₂₀, -O-(C₁-C₁₂-Alkylen)-OR₁₇, -(C₁-C₁₂-Alkylen)-O-CO-R₁₇, -(C₁-C₁₂-Alkylen)-OR₁₇, -COOR₁₇, -(C₁-C₁₂-Alkylen)-COOR₁₇, -(C₁-C₁₂-Alkylen)-CONR₁₉R₂₀, -(C₁-C₁₂-Alkylen)-COOX, -COOX, -COOH,-SR₁₇, -SOR₁₇, -SO₂R₁₇, -SO₂NR₁₉R₂₀, -SO₃R₁₇ oder SO₃X, SO₃H wobei die Reste R₁₇, R₁₈, R₁₉, R₂₀ die weiter oben angegebene Bedeutung haben, und
R₂₁ unabhängig von R₁ die gleiche Bedeutung wie R₁ besitzt, und
A₃ eine Einfachbindung, oder unsubstituiertes oder einfach oder mehrfach mit C₁-C₂₅Alkyl, C₆-C₂₄Aryl, Halogen, Hydroxy, -OX, Oxo, Cyano, -COOR₆, -COOX, -COOH, -SO₃R₆, -SO₃X, -SO₃H substituiertes C₁-C₂₄-Alkylen oder C₅-C₁₂-Cycloalkylen oder -OOC-(C₁-C₂₄-Alkylen)-COO-, -COO-(C₁-C₂₄-Alkylen)-OOC-, -NR₁₉CO-(C₁-C₂₄-Alkylen)-CONR₁₉-, -CONR₁₉-(C₁-C₂₄-Alkylen)-NR₁₉CO-; C₆-C₂₄Arylen oder Heteroarylen, bedeutet.

5. Oxobenzofuranyliden-Dihydroindolone gemäss einem der Ansprüche 1 oder 2, **dadurch gekennzeichnet, dass** A₁ für 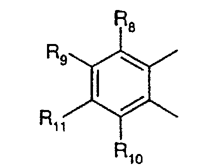 wobei R₈, R₉, R₁₁ und R₁₀ die in Anspruch 3 angegebene Bedeutung haben, und A₂ für einen zweiwertigen Rest Z₅ oder Z₆ 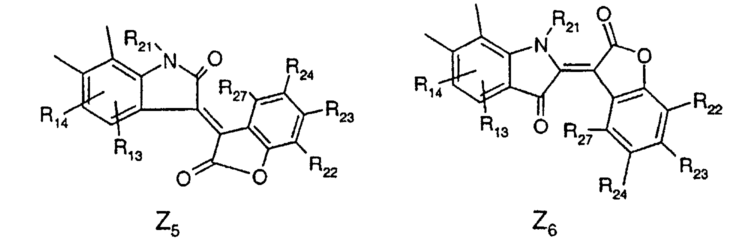 steht, worin R₁₃, R₁₄, R₂₁, R₂₂, R₂₃, R₂₄ und R₂₇ die in Anspruch 4 angegebene Bedeutung haben.

6. Oxobenzofuranyliden-Dihydroindolone gemäss Anspruch 1, **dadurch gekennzeichnet, dass** sie für eine Verbindung der Formel (LI) 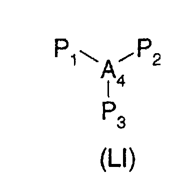 worin
P₁, P₂, P₃ unabhängig voneinander für jeweils einen Rest, ausgewählt aus der Gruppe, bestehend aus den Resten Z₁, Z₂, Z₃ und Z₄ gemäss Anspruch 4, und
A₄ für einen trivalenten Rest stehen.

7. Oxobenzofuranyliden-Dihydroindolone (LI) gemäss Anspruch 6, **dadurch gekennzeichnet, dass** der trivalente Rest für 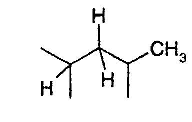 steht.

8. Cis und trans Bisbenzofuranon-Derivate der Oxobenzofuranyliden-Dihydroindolone gemäss einem der Ansprüche 1 oder 3, **dadurch gekennzeichnet, dass** sie für Verbindungen der Formeln 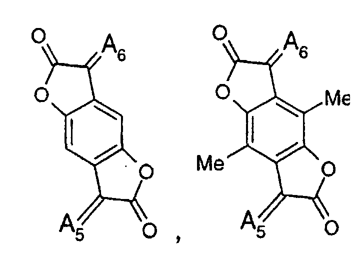 stehen,
worin
A₅ und A₆ unabhängig voneinander für jeweils zwei Wasserstoffe stehen, unter der Massgabe, dass sie nicht gleichzeitig für zwei Wasserstoffe stehen; des weiteren stehen sie unabhängig voneinander für einen substituierten oder unsubstituierten Isatin-Rest 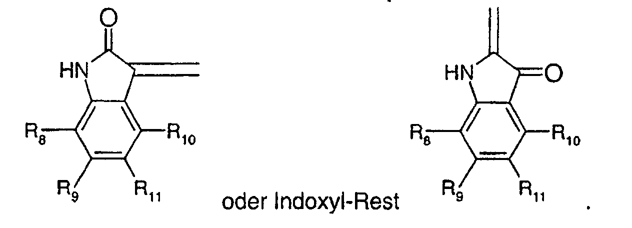 bedeuten.

9. Zusammensetzungen, enthaltend mindestens ein Oxobenzofuranyliden-Dihydroindolon gemäss einem der Ansprüche 1 bis 8.

10. Verfahren zur Herstellung der Oxobenzofuranyliden-Dihydroindolone der Formeln trans-(Ia) und cis-(Ib) gemäss Anspruch 1 durch Umsetzung eines Benzofuranon-oder Bisbenzofuranon-Derivats mit einem Isatin-Derivat in Gegenwart eines sauren oder basischen Katalysators, **dadurch gekennzeichnet, dass** man eine Verbindung der Formel (XXIX) 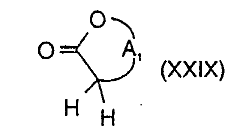 in der A₁ die in Anspruch 1 angegebene Bedeutung hat, mit einer Verbindung der Formel (XXX) oder (XXXa), 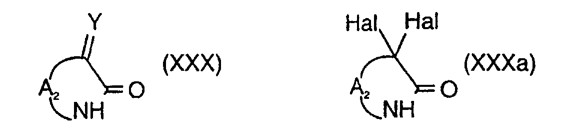 worin
Y für O, S, NH, N-(C₁-C₂₄Alkyl), N-(C₅-C₁₈Aryl) oder N-(C₇-C₂₈Aralkyl) und
Hal für Halogen stehen, und
A₂ die in Anspruch 1 angegebene Bedeutung hat, umsetzt.

11. Verfahren zur Herstellung der Oxobenzofuranyliden-Dihydroindolone der Formeln trans-(Ia) und cis-(Ib) gemäss Anspruch 1 durch Umsetzung eines Benzofuranon- oder Bisbenzofuranon-Derivats mit einem Oxindol-Derivat in Gegenwart eines sauren oder basischen Katalysators, **dadurch gekennzeichnet, dass** man eine Verbindung der Formel (XXXI) oder (XXXIa) 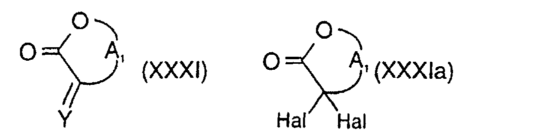 in der A₁ die in Anspruch 1,Y und Hal die in Anspruch 10 angegebenen Bedeutungen haben, mit einer Verbindung der Formel (XXXII) 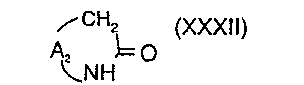 in der A₂ die in Anspruch 1 angegebene Bedeutung hat, umsetzt.

12. Verfahren zur Herstellung von Verbindungen der Formeln trans-(IIa) und cis-(IIb) gemäss Anspruch 1, durch Umsetzung eines Benzofuranon- oder Bisbenzofuranon-Derivats mit einem Indoxyl-Derivat in Gegenwart einer Säure oder einer Base, **dadurch gekennzeichnet, dass** man eine Verbindung der Formeln (XXXI) oder (XXXIa) mit einer Verbindung der Formel (XXXIV) 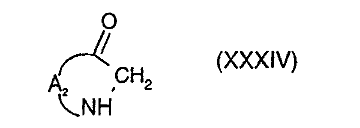 worin A₂ die in Anspruch 1 angegebene Bedeutung hat, umsetzt.

13. Verfahren zur Herstellung von Verbindungen der Formeln trans-(Ia), trans-(IIa) oder cis-(Ib), cis-(IIb) gemäss Anspruch 1 durch Umsetzung eines Benzofuranon- oder Bisbenzofuranon-Derivats mit einem Indoxyl- oder Isatin-Derivat in Gegenwart einer Säure oder einer Base, **dadurch gekennzeichnet, dass** man eine Verbindung der Formel (XXXI) oder (XXXIa) nach Anspruch 11 mit
(a) einem Imin der Formel (XXXV) 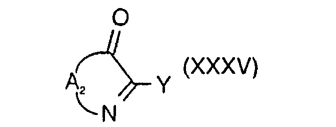 in der A₂ und Y die in Anspruch 1 und 10 angegebenen Bedeutungen haben, oder
(b) einem Ketal der Formeln (XXXVI) oder (XXXVII) 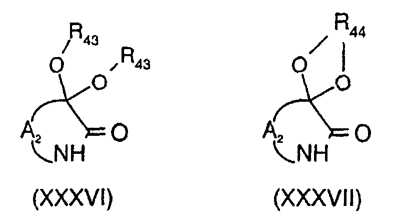
worin
R₄₃ C₁-C₆Alkyl und R₄₄ einen unsubstituierten oder substituierten bivalenten C₁-C₆-Alkylen-Rest bedeuten,
umsetzt.

14. Verwendung der Oxobenzofuranyliden-Dihydroindolone gemäss einem der Ansprüche 1 bis 7 oder hergestellt nach einem Verfahren der Ansprüche 10 bis 13 sowie der Zusammensetzung nach Anspruch 9 zur Färbung oder Pigmentierung von organischen oder anorganischen Materialien, insbesondere zur Herstellung von Tinten oder Farbmitteln für Lacke, Druckfarben, Mineralöle, Schmierfette oder Wachse, gefärbten oder pigmentierten Kunststoffen, non-impact-printing-Material, Farbfiltern, Kosmetikartikeln, Tonern oder zur Einfärbung von Holzbeizen.

## Claims

1. An oxobenzofuranylidene-dihydroindolone of formula trans-(Ia) or cis-(Ib) 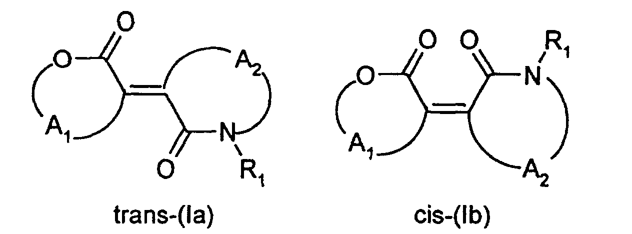 or of formula trans-(IIa) or cis-(IIb) 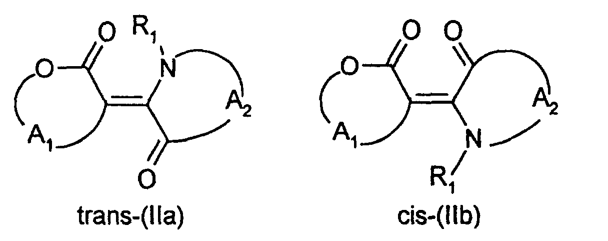 wherein
A₁ and A₂ are each independently of the other unsubstituted or mono- to tetra-substituted ortho-C₆-C₁₈arylene, and
R₁ is hydrogen, C₁-C₂₅alkyl, C₅-C₁₂cycloalkyl, C₆-C₂₄aryl, a heteroaromatic radical, -(CH₂)ₙ-COR₂ or -(CH₂)ₘ-OR₃,
wherein R₂ is hydroxy, -OX, or, unsubstituted or mono- or poly-substituted by hydroxy, -OX or by amino, C₁-C₂₅alkoxyl C₁-C₂₅alkylamino or C₁-C₂₅dialkylamino, di(C₆-C₂₄aryl)amino, C₁-C₁₂alkyl, C₂-C₂₄alkenyl, and
X is a cation, and
R₃ is hydrogen or -CO-(C₁-C₂₅alkyl), and
n and m denote each independently of the other a whole number in the range from 0 to 6, and
a single C-C bond also may have been replaced by a corresponding ether unit, C-O-C,
with the proviso that, when R₁ is hydrogen and A₂ is 1,2-phenylene, A₁ is not 9,10-anthraquinon-1,2-ylene, 4-chloro-3,5-dimethyl-1,2-phenylene or 3,5-dimethyl-1,2-phenylene.

2. An oxobenzofuranylidene-dihydroindolone according to claim 1, wherein A₁ and/or A₂ are/is substituted or unsubstituted 1,2-phenylene, 1,2-naphthylene, 2,3-naphthylene, 1,2-phenanthrylene, 2,3-phenanthrylene, 3,4-phenanthrylene, 9,10-phenanthrylene, 1,2-anthracenyl, 2,3-anthracenyl or 1,2-anthraquinonylene or 2,3-anthraquinonylene.

3. An oxobenzofuranylidene-dihydroindolone according to either claim 1 or claim 2, wherein A₁ is 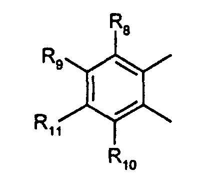 and A₂ is 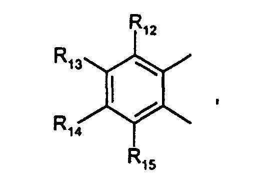 wherein
R₈, R₉, R₁₁, R₁₀, R₁₂, R₁₃, R₁₄ and R₁₅ are each independently of the others hydrogen, halogen, cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂alkylene)-COOX, -O-(C₁-C₁₂alkylene)-COOH, -O-(C₁-C₁₂alkylene)-O-CO-R₁₇, -O-(C₁-C₁₂alkylene)-COOR₁₇, -O-(C₁-C₁₂alkylene)-CONR₁₉R₂₀, -O-(C₁-C₁₂alkylene)-OR₁₇, -(C₁-C₁₂alkylene)-O-CO-R₁₇, -(C₁-C₁₂alkylene)-OR₁₇, -COOR₁₇, -(C₁-C₁₂alkylene)-COOR₁₇, -(C₁-C₁₂alkylene)-CONR₁₉R₂₀, -(C₁-C₁₂alkylene)-COOX, -(C₁-C₁₂alkylene)-COOH, -COOX, -COOH, -SR₁₇, -SOR₁₇, -SO₂R₁₇, -SO₂NR₁₉R₂₀, -SO₃R₁₇, SO₃H or SO₃X,
wherein
R₁₇, R₁₈, R₁₉ and R₂₀ are each independently of the others hydrogen or R₁₆, and
R₁₆ is, unsubstituted or mono- or poly-substituted by halogen, hydroxy, amino, oxo, carboxy, cyano, -COOR₁₈ or by -COOX, C₁-C₂₅alkyl, C₅-C₁₂cycloalkyl or C₂-C₂₄alkenyl, which may be uninterrupted or interrupted one or more times by O, S or N-(C₁-C₂₅alkyl), N-(C₂-C₂₄alkenyl) when the alkyl has more than two and the alkenyl more than three carbon atoms, or is, unsubstituted or mono- or poly-substituted by halogen, nitro, cyano, -OR₁₈, -SR₁₈, -NR₁₉R₂₀, -CONR₁₉R₂₀, -COOR₁₈, -COOX, -COOH, -SO₂R₁₈, -SO₂NR₁₉R₂₀, -SO₃R₁₈, -SO₃X, -SO₃H, -NR₁₉COR₁₈ or by -NR₁₉COOR₁₈, C₆-C₁₈aryl, C₇-C₁₈aralkyl or heteroaryl,
or
R₁₉ and R₂₀, together with the nitrogen atom linking them, are unsubstituted or mono-to tetra-C₁-C₄alkyl-substituted pyrrolidinyl, piperidyl, piperazinyl or morpholinyl, or are carbazolyl, phenoxazinyl or phenothiazinyl,
it being possible, optionally, for R₈ and R₉, R₉ and R₁₁, R₁₁ and R₁₀ and for R₁₂ and R₁₃, R₁₃ and R₁₄ or R₁₄ and R₁₅ additionally to form, in each case, a substituted or unsubstituted 5-or 6-membered ring.

4. An oxobenzofuranylidene-dihydroindolone according to claim 3, wherein at least one of the radicals R₈, R₉, R₁₁, R₁₀, R₁₂, R₁₃, R₁₄ and R₁₅ is a radical selected from the group consisting of the radicals Z₁, Z₂, Z₃ and Z₄ 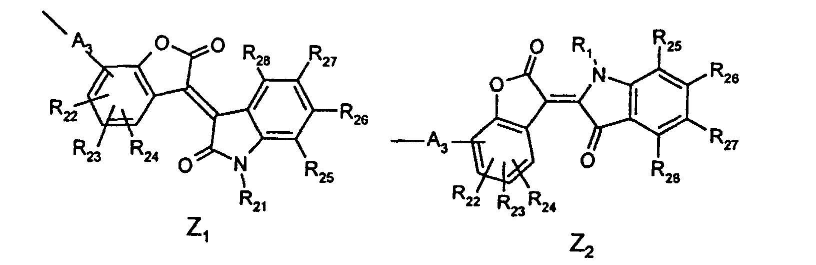 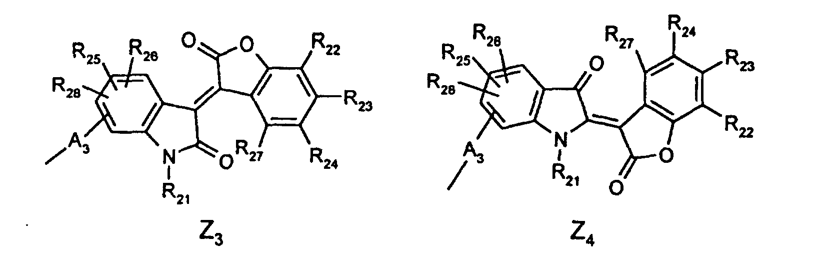 wherein
R₂₂, R₂₃, R₂₄, R₂₅, R₂₆, R₂₇ and R₂₈ are each independently of the others hydrogen, halogen, cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(C₁-C₁₂alkylene)-COOX, -O-(C₁-C₁₂alkylene)-COOH, -O-(C₁-C₁₂alkylene)-O-CO-R₁₇, -O-(C₁-C₁₂alkylene)-COOR₁₇, -O-(C₁-C₁₂alkylene)-CONR₁₉R₂₀, -O-(C₁-C₁₂alkylene)-OR₁₇, -(C₁-C₁₂alkylene)-O-CO-R₁₇, -(C₁-C₁₂alkylene)-OR₁₇, -COOR₁₇, -(C₁-C₁₂alkylene)-COOR₁₇, -(C₁-C₁₂alkylene)-CONR₁₉R₂₀, -(C₁-C₁₂alkylene)-COOX, -COOX, -COOH, -SR₁₇, -SOR₁₇, -SO₂R₁₇, -SO₂NR₁₉R₂₀, -SO₃R₁₇ or SO₃X, SO₃H, the radicals R₁₇, R₁₈, R₁₉, R₂₀ being as defined above, and
R₂₁, independently of R₁, has the same definition as R₁, and
A₃ is a single bond or is, unsubstituted or mono- or poly-substituted by C₁-C₂₅alkyl, C₆-C₂₄aryl, halogen, hydroxy, -OX, oxo, cyano, -COOR₆, -COOX, -COOH, -SO₃R₆, -SO₃X or by -SO₃H, C₁-C₂₄alkylene or C₅-C₁₂cycloalkylene or -OOC-(C₁-C₂₄alkylene)-COO-, -COO-(C₁-C₂₄alkylene)-OOC-, -NR₁₉CO-(C₁-C₂₄alkylene)-CONR₁₉-, -CONR₁₉-(C₁-C₂₄alkylene)-NR₁₉CO-; C₆-C₂₄arylene or heteroarylene.

5. An oxobenzofuranylidene-dihydroindolone according to either claim 1 or claim 2, wherein A₁ is 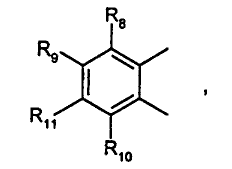 wherein R₈, R₉, R₁₁ and R₁₀ are as defined in claim 3,
and A₂ is a bivalent radical Z₅ or Z₆ 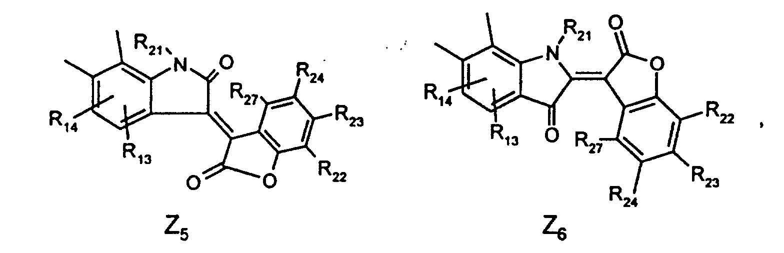 wherein R₁₃, R₁₄, R₂₁, R₂₂, R₂₃, R₂₄ and R₂₇ are as defined in claim 4.

6. An oxobenzofuranylidene-dihydroindolone according to claim 1, which is a compound of formula (LI) 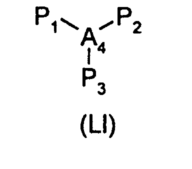 wherein
P₁, P₂, P₃ are each independently of the others a radical selected from the group consisting of the radicals Z₁, Z₂, Z₃ and Z₄ according to claim 4, and
A₄ is a trivalent radical.

7. An oxobenzofuranylidene-dihydroindolone (LI) according to claim 6, wherein the trivalent radical is

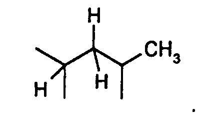

8. A cis- or trans-bisbenzofuranone derivative of an oxobenzofuranylidene-dihydroindolone according to either claim 1 or claim 3, which is a compound of formula 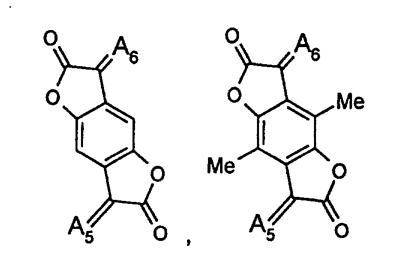 wherein
A₅ and A₆ denote each independently of the other two hydrogen atoms, with the proviso that they do not simultaneously denote two hydrogen atoms; they furthermore denote each independently of the other a substituted or unsubstituted isatin radical

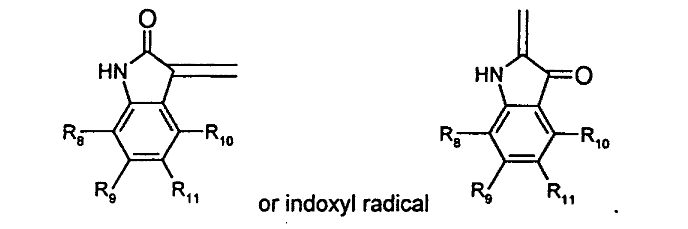

9. A composition comprising at least one oxobenzofuranylidene-dihydroindolone according to any one of claims 1 to 8.

10. A process for the preparation of an oxobenzofuranylidene-dihydroindolone of formula trans-(Ia) or cis-(Ib) according to claim 1 by reaction of a benzofuranone or bisbenzofuranone derivative with an isatin derivative in the presence of an acidic or basic catalyst, which process comprises reacting a compound of formula (XXIX) 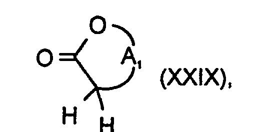 wherein A₁ is as defined in claim 1, with a compound of formula (XXX) or (XXXa) 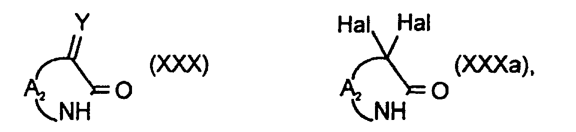 wherein
Y is O, S, NH, N-(C₁-C₂₄alkyl), N-(C₅-C₁₈aryl) or N-(C₇-C₂₈aralkyl) and
Hal is halogen, and
A₂ is as defined in claim 1.

11. A process for the preparation of an oxobenzofuranylidene-dihydroindolone of formula trans-(Ia) or cis-(Ib) according to claim 1 by reaction of a benzofuranone or bisbenzofuranone derivative with an oxindole derivative in the presence of an acidic or basic catalyst, which process comprises reacting a compound of formula (XXXI) or (XXXIa) 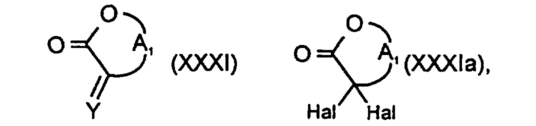 wherein A₁ is as defined in claim 1 and Y and Hal are as defined in claim 10, with a compound of formula (XXXII) 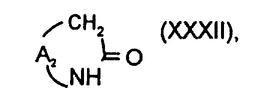 wherein A₂ is as defined in claim 1.

12. A process for the preparation of a compound of formula trans-(IIa) or cis-(IIb) according to claim 1 by reaction of a benzofuranone or bisbenzofuranone derivative with an indoxyl derivative in the presence of an acid or a base, which process comprises reacting a compound of formula (XXXI) or (XXXIa) with a compound of formula (XXXIV) 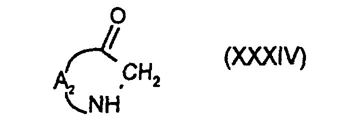 wherein A₂ is as defined in claim 1.

13. A process for the preparation of a compound of formula trans-(Ia), trans-(IIa) or cis-(Ib), cis-(IIb) according to claim 1 by reaction of a benzofuranone or bisbenzofuranone derivative with an indoxyl or isatin derivative in the presence of an acid or a base, which process comprises reacting a compound of formula (XXXI) or (XXXIa) according to claim 11 with
(a) an imine of formula (XXXV) 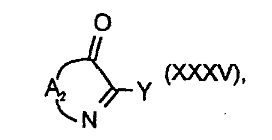 wherein A₂ and Y are as defined in claim1 and claim 10,
or
(b) a ketal of formula (XXXVI) or (XXXVII) 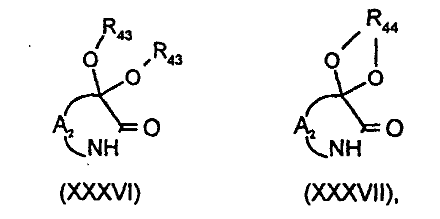
wherein
R₄₃ is C₁-C₆alkyl and R₄₄ is an unsubstituted or substituted bivalent C₁-C₆alkylene radical.

14. Use of an oxobenzofuranylidene-dihydroindolone according to any one of claims 1 to 7 or prepared according to a process of claims 10 to 13 and of a composition according to claim 9 in the colouring or pigmenting of organic or inorganic materials, especially in the production of inks or colourants for surface-coatings, printing inks, mineral oils, lubricating greases or waxes, coloured or pigmented plastics, non-impact-printing material, colour filters, cosmetic articles, toners or in the colouring of woodstains.

## Revendications

1. Oxobenzofuranylidène-dihydroindolones de formules trans-(Ia) et cis-(Ib) 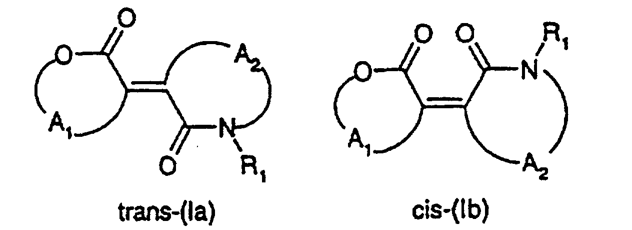 ainsi que de formules trans-(IIa) et cis-(IIb) 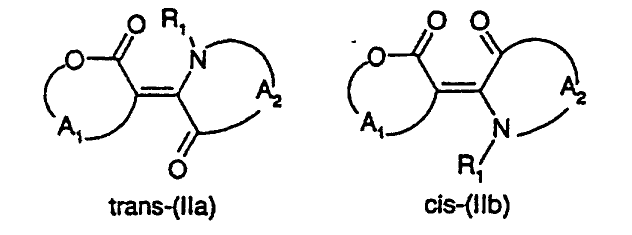 où
A₁ et A₂ représentent, indépendamment l'un de l'autre, un groupe ortho-arylène en C₆-C₁₈ non substitué ou substitué 1 à 4 fois, et
R₁ représente un atome d'hydrogène, des groupes alkyle en C₁-C₂₅, cycloalkyle en C₅-C₁₂, aryle en C₆-C₂₄, un reste hétéroaromatique, un groupe -(CH₂)ₙ-COR₂ ou -(CH₂)ₘ-OR₃, où
R₂ représente des groupes hydroxy, -OX, alkoxy en C₁-C₂₅, non substitué ou substitué une ou plusieurs fois par des substituants hydroxy, -OX ou amino, (alkyl en C₁-C₂₅)amino ou di-(alkyl en C₁-C₂₅)amino, di-(aryl en C₆-C₂₄)amino, alkyle en C₁-C₁₂, alcényle en C₂-C₂₄, et
X représente un cation, et
R₃ représente un atome d'hydrogène ou un groupe -CO-(alkyle en C₁-C₂₅), et
n et m sont, indépendamment l'un de l'autre, un entier dans le domaine de 0 à 6, et
une simple liaison C-C peut aussi être remplacée par une unité éther correspondante, C-O-C,
à condition que lorsque R₁ représente un atome d'hydrogène et A₂ représente un groupe 1,2-phénylène, A₁ ne représente pas un groupe 9,10-anthraquinon-1,2-ylène, 4-chloro-3,5-diméthyl-1,2-phénylène ou 3,5-diméthyl-1,2-phénylène.

2. Oxobenzofuranylidène-dihydroindolones selon la revendication 1, **caractérisées en ce que** A₁ et/ou A₂ représentent des groupes 1,2-phénylène, 1,2-naphtylène, 2,3-naphtylène, 1,2-phénanthrylène, 2,3-phénanthrylène, 3,4-phénanthrylène, 9,10-phénanthrylène, 1,2-anthracényle, 2,3-anthracényle ou 1,2-anthraquinonylène ou 2,3-anthraquinonylène substitués ou non substitués.

3. Oxobenzofuranylidène-dihydroindolones selon l'une des revendications 1 ou 2, **caractérisées en ce que** A₁ représente 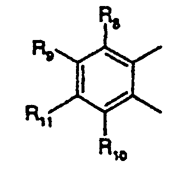 et A₂ représente 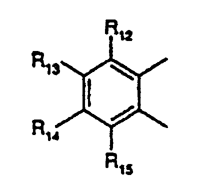 où
R₈, R₉, R₁₁, R₁₀, R₁₂, R₁₃, R₁₄ et R₁₅ représentent, indépendamment les uns des autres, des atomes d'hydrogène, des atomes d'halogènes, des groupes cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(alkylène en C₁-C₁₂)-COOX, -O-(alkylène en C₁-C₁₂)-COOH, -O-(alkylène en C₁-C₁₂)-O-CO-R₁₇, -O-(alkylène en C₁-C₁₂)-COOR₁₇, -O-(alkylène en C₁-C₁₂)-CO-NR₁₉R₂₀, -O-(alkylène en C₁-C₁₂)-OR₁₇, -(alkylène en C₁-C₁₂)-O-CO-R₁₇, -(alkylène en C₁-C₁₂)-O-R₁₇, -COOR₁₇, -(alkylène en C₁-C₁₂)-COOR₁₇, -(alkylène en C₁-C₁₂)-CONR₁₉R₂₀, -(alkylène en C₁-C₁₂)-COOX, -(alkylène en C₁-C₁₂)-COOH, -COOX, -COOH, -SR₁₇, -SOR₁₇, -SO₂R₁₇, -SO₂NR₁₉R₂₀, -SO₃R₁₇, -SO₃H ou -SO₃X,
où
R₁₇, R₁₈, R₁₉ et R₂₀ représentent, indépendamment les uns des autres, des atomes d'hydrogène ou un groupe R₁₆, et
R₁₆ représente un groupe alkyle en C₁-C₂₅ non substitué ou substitué une ou plusieurs fois par des substituants halogène, hydroxy, amino, oxo, carboxy, cyano, -COOR₁₈ ou -COOX, cycloalkyle en C₂-C₁₂ ou alcényle en C₂-C₂₄ qui peut être non interrompu ou interrompu une ou plusieurs fois par groupes O, S ou N-(alkyle en C₁-C₂₅), N-(alcényle en C₂-C₂₄), dans le cas où le groupe alkyle présente plus de deux et le groupe alcényle plus de trois atomes de carbone, ou représente des groupes aryle en C₆-C₁₈, aralkyle en C₇-C₁₈ ou hétéroaryle non substitué ou substitué une ou plusieurs fois par des substituants halogène, nitro, cyano, -OR₁₈, -SR₁₈, -NR₁₉R₂₀, -CONR₁₉R₂₀, -COOR₁₈, -COOX, -COOH, -SO₂R₁₈, -SO₂NR₁₉R₂₀, -SO₃R₁₈, -SO₃X, -SO₃H, -NR₁₉COR₁₈ ou -NR₁₉COOR₁₈,
ou
R₁₉ et R₂₀ représentent ensemble avec l'atome d'azote qui les relie, un groupe pyrrolidinyle, pipéridinyle, pipérazinyle ou morpholinyle, carbazolyle, phénoxazinyle ou phénothiazinyle non substitué ou substitué une à quatre fois par un substituant alkyle en C₁-C₄,
éventuellement chacun des R₈ et R₉, R₉ et R₁₁, R₁₁ et R₁₀, ainsi que R₁₂ et R₁₃, R₁₃ et R₁₄ ou R₁₄ et R₁₅ peuvent former de plus un cycle substitué ou non substitué à 5 ou 6 chaînons.

4. Oxobenzofuranylidène-dihydroindolones selon la revendication 3, **caractérisées en ce que** au moins un des restes R₈, R₉, R₁₁, R₁₀, R₁₂, R₁₃, R₁₄ et R₁₅ représentent un reste pris dans le groupe comprenant les restes
Z₁, Z₂, Z₃ et Z₄ 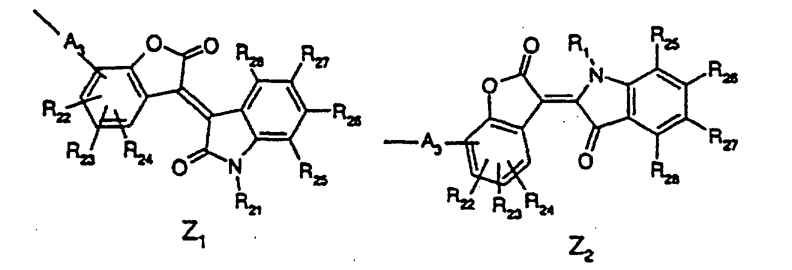 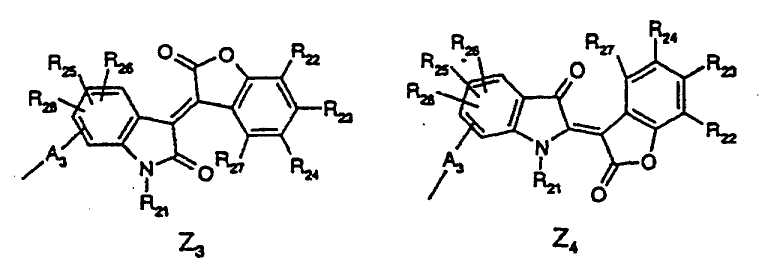 où
R₂₂, R₂₃, R₂₄, R₂₅, R₂₆, R₂₇ et R₂₈ représentent, indépendamment les uns des autres, des atomes d'hydrogène, des atomes d'halogènes, des groupes cyano, -NO₂, -R₁₆, -NR₁₇R₁₈, -NR₁₉COR₁₇, -NR₁₉COOR₁₇, -N=CR₁₇R₁₈, -CONR₁₉R₂₀, -OR₁₇, -O-(alkylène en C₁-C₁₂)-COOX, -O-(alkylène en C₁-C₁₂)-COOH, -O-(alkylène en C₁-C₁₂)-O-CO-R₁₇, -O-(alkylène en C₁-C₁₂)-COOR₁₇, -O-(alkylène en C₁-C₁₂)-CONR₁₉R₂₀, -O-(alkylène en C₁-C₁₂)-OR₁₇, -(alkylène en C₁-C₁₂)-O-CO-R₁₇, -(alkylène en C₁-C₁₂)-OR₁₇, -COOR₁₇, -(alkylène en C₁-C₁₂)-COOR₁₇, -(alkylène en C₁-C₁₂)-CONR₁₉R₂₀, -(alkylène en C₁-C₁₂)-COOX, -COOX, -COOH, -SR₁₇, -SOR₁₇, -SO₂R₁₇, -SO₂NR₁₉R₂₀, -SO₃R₁₇ ou -SO₃X, -SO₃H, les restes R₁₇, R₁₈, R₁₉, R₂₀ possèdent les significations données ci-dessus, et
R₂₁ possède indépendamment de R₁, la même signification que R₁, et
A₃ représente une simple liaison, ou des groupes-CONR₁₉-(alkylène en C₁-C₂₄)-NR₁₉CO-, -NR₁₉CO-(alkylène en C₁-C₂₄)-CONR₁₉-, -COO-(alkylène en C₁-C₂₄)-OOC-, -OOC-(alkylène en C₁-C₂₄)-COO-, cycloalkylène en C₅-C₁₂ ou alkylène en C₁-C₂₄ non substitué ou substitué une ou plusieurs fois par des substituants alkyle en C₁-C₂₅, aryle en C₆-C₂₄, halogène, hydroxy, -OX, oxo, cyano, -XOOR₆, COOX, -COOS, -SO₃R₆, -SO₃X, -SO₃H ; ou arylène en C₆-C₂₄ ou hétéroarylène.

5. Oxobenzofuranylidène-dihydroindolones selon l'une des revendications 1 ou 2, **caractérisées en ce que** A₁ représente 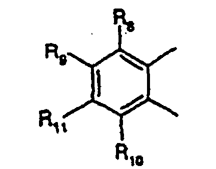 où
R₈, R₉, R₁₁ et R₁₀ possèdent la signification donnée à la revendication 3, et
A₂ représente un reste bivalent Z₅ ou Z₆ 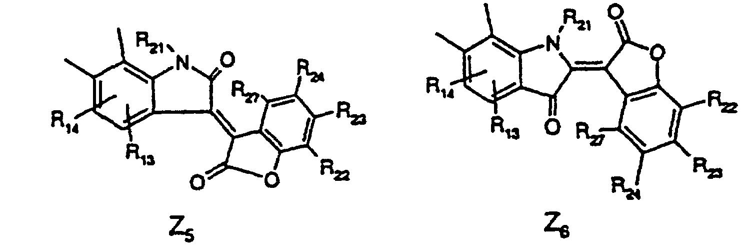
où R₁₃, R₁₄, R₂₁, R₂₂, R₂₃, R₂₄ et R₂₇ possèdent la signification donnée à la revendication 4.

6. Oxobenzofuranylidène-dihydroindolones selon la revendication 1, **caractérisées en ce qu'**elles représentent un composé de formule (LI) 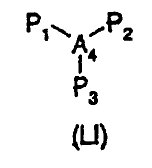 où
P₁, P₂, P₃ représentent chacun, indépendamment les uns des autres, un reste pris dans le groupe comprenant les restes Z₁, Z₂, Z₃ et Z₄ selon la revendication 4, et
A₄ représente un reste trivalent.

7. Oxobenzofuranylidène-dihydroindolones (LI) selon la revendication 6, **caractérisées en ce que** le reste trivalent représente

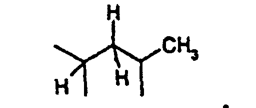

8. Dérives cis et trans de la bisbenzofuranone des oxobenzofuranylidène-dihydroindolones selon l'une des revendications 1 ou 3, **caractérisés en ce qu'**ils représentent des composés de formules 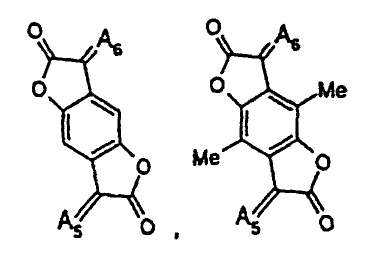 où
A₅ et A₆ représentent chacun, indépendamment l'un de l'autre, deux atomes d'hydrogène, à condition qu'ils ne représentent pas simultanément deux atomes d'hydrogène ; de plus, ils représentent, indépendamment l'un de l'autre, un reste isatine non substitué ou substitué

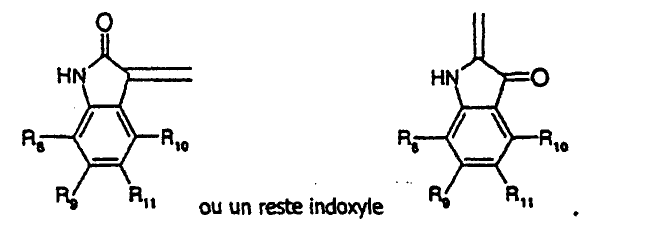

9. Compositions contenant au moins une oxobenzofuranylidène-dihydroindolone selon l'une des revendications 1 à 8.

10. Procédé pour la préparation des oxobenzofuranylidène-dihydroindolones des formules trans(Ia) et cis-(Ib) selon la revendication 1, par réaction d'un dérivé de la benzofuranone ou bisbenzofuranone avec un dérivé de l'isatine en présence d'un catalyseur acide ou basique, **caractérisé en ce qu'**on fait réagir un composé de formule (XXIX) 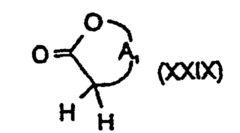 où A₁ possède la signification donnée ci-dessus, avec un composé de formule (XXX) ou (XXXa) 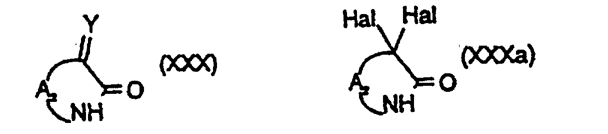 où
Y représente un groupe O, S, NH, N-(alkyle en C₁-C₂₄), N-(aryle en C₅-C₁₈) ou N-(aralkyle en C₇-C₂₈) et
Hal représente un atome d'halogène, et
A₂ possède la signification donnée à la revendication 1.

11. Procédé pour la préparation des oxobenzofuranylidène-dihydroindolones des formules trans-(Ia) et cis-(Ib) selon la revendication 1, par réaction d'un dérivé de la benzofuranone ou bisbenzofuranone avec un dérivé de l'oxindole en présence d'un catalyseur acide ou basique, caractérusé en ce qu'on fait réagir un composé de formule (XXXI) ou (XXXIa) 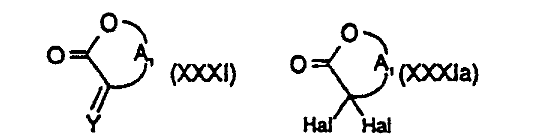 où A₁ possède la signification donnée à la revendication 1, Y et Hal possèdent les significations données à la revendication 10, avec un composé de formule (XXXII) 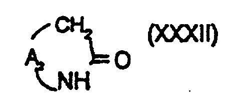 où A₂ possède la signification donnée à la revendication 1.

12. Procédé pour la préparation de composés de formules trans-(IIa) et cis-(IIb) selon la revendication 1, par transformation d'un dérivé de la benzofuranone ou bisbenzofuranone avec un dérivé de l'indoxyle en présence d'un acide ou d'une base, **caractérisé en ce qu'**on transforme un composé de formules (XXXI) ou (XXXIa) avec un composé de formule (XXXIV) 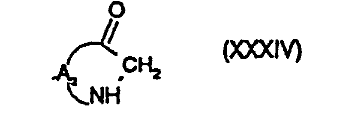 où A₂ possède la signification donnée à la revendication 1.

13. Procédé pour la préparation de composés de formules trans-(Ia), trans-(IIa) ou cis-(Ib), cis-(IIb) selon la revendication 1, par transformation d'un dérivé de la benzofuranone ou bisbenzofuranone avec un dérivé de l'indoxyle ou de l'isatine en présence d'un acide ou d'une base, **caractérisé en ce qu'**on transforme un composé de formules (XXXI) ou (XXXIa) selon la revendication 11
(a) avec une imine de formule (XXXV) 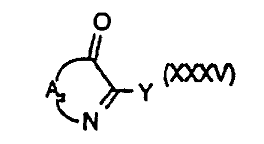 et A₂ et Y possèdent les significations données à la revendication 1 et 10,
ou
(b) avec un cétal de formules (XXXVI) ou (XXXVII) 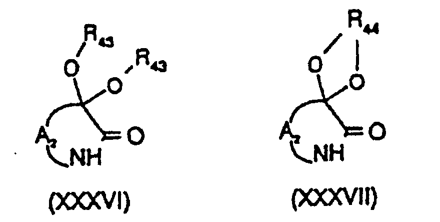
où
R₄₃ représente un groupe alkyle en C₁-C₆ et
R₄₄ représente un reste alkylène en C₁-C₆ bivalent non substitué ou substitué.

14. Utilisation des oxobenzofuranylidène-dihydroindolones selon l'une des revendications 1 à 7 ou préparés selon un procédé des revendications 10 à 13 ainsi que de la composition selon la revendication 9 pour la teinture ou la pigmentation de matières organiques ou inorganiques, en particulier pour la préparation d'encres ou de colorants pour vernis, encres d'imprimerie, huiles minérales, graisses ou cires, matières synthétiques teintes ou pigmentées, matières d'impression mécanique, filtres colorés, produits cosmétiques, toners ou pour le mordançage du bois.
